(19) Europäisches Patentamt / European Patent Office / Office européen des brevets

(11) **EP 3 564 391 B1**

(12) **EUROPEAN PATENT SPECIFICATION**

(45) Date of publication and mention
of the grant of the patent:
**24.11.2021 Bulletin 2021/47**

(51) Int Cl.:
*C12Q 1/68* *(2018.01)*          *G16B 20/10* *(2019.01)*
*G16B 20/20* *(2019.01)*

(21) Application number: **17888263.5**

(22) Date of filing: **25.12.2017**

(86) International application number:
**PCT/CN2017/118213**

(87) International publication number:
**WO 2018/121468 (05.07.2018 Gazette 2018/27)**

(54) **METHOD, DEVICE AND KIT FOR DETECTING FETAL GENETIC MUTATION**

VERFAHREN, VORRICHTUNG UND KIT ZUM NACHWEIS VON FETALER GENETISCHER MUTATION

PROCÉDÉ, DISPOSITIF ET KIT POUR LA DÉTECTION D'UNE MUTATION GÉNÉTIQUE CHEZ UN FOETUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2016 CN 201611270836**

(43) Date of publication of application:
**06.11.2019 Bulletin 2019/45**

(73) Proprietor: **Annoroad Gene Technology (Beijing) Co., Ltd**
**Beijing 100176 (CN)**

(72) Inventors:
• **DU, Yang**
  **Beijing 100176 (CN)**
• **PENG, Shengbin**
  **Beijing 100176 (CN)**
• **HUI, Feng**
  **Beijing 100176 (CN)**
• **ZHANG, Han**
  **Beijing 100176 (CN)**
• **XUAN, Zhaoling**
  **Beijing 100176 (CN)**
• **LI, Dawei**
  **Beijing 100176 (CN)**
• **LIANG, Junbin**
  **Beijing 100176 (CN)**
• **CHEN, Chongjian**
  **Beijing 100176 (CN)**

(74) Representative: **Maiwald Patent- und Rechtsanwaltsgesellschaft mbH**
**Elisenhof**
**Elisenstraße 3**
**80335 München (DE)**

(56) References cited:
**WO-A1-2008/088804     WO-A1-2016/183106**
**AU-A1- 2015 249 846   CN-A- 103 874 767**
**US-A1- 2013 123 120**

Sequence Listing

<110> Annoroad Gene Technology (Beijing) Co., Ltd.

<120> Method, device and kit for detecting fetal gene mutation

<130> A16840WOEP/MH

<140> EP 17 888 263.5

<141> 25.12.2017

<150> CN201611270836.9

<151> 29.12.2016

<150> PCT/CN2017/118213

<151> 25.12.2017

<160> 8

<170> PatentIn version 3. 5

<210> 1

<211> 20

<212> DNA

<213> Artificial Sequence

<220>

<223> Adapter sequence 1 in library construction

<400> 1

gatcggaaga gcacacgtct 20

<210> 2

<211> 33

<212> DNA

<213> Artificial Sequence

<220>

<223> Adapter sequence 2 in library construction

<400> 2

acactctttc cctacacgac gctcttccga tct 33

<210> 3

<211> 58

<212> DNA

<213> Artificial Sequence

<220>

<223> primer 1 for amplification

<400> 3

aatgatacgg cgaccaccga gatctacact ctttccctac acgacgctct tccgatct 58

<210> 4

<211> 64

<212> DNA

<213> Artificial Sequence

<220>

<223> primer 2 for amplification

<400> 4

```
caagcagaag acggcatacg agatcgtgat gtgactggag ttcagacgtg tgctcttccg     60
atct                                                                  64
```

<210> 5

<211> 58

<212> DNA

<213> Artificial Sequence

<220>

<223> blocking primer 1 for exon trapping

<400> 5

aatgatacgg cgaccaccga gatctacact ctttccctac acgacgctct tccgatct 58

<210> 6

<211> 64
<212> DNA
<213> Artificial Sequence
<220>
<223> blocking primer 2 for exon trapping
<400> 6

```
caagcagaag acggcatacg agatcgtgat gtgactggag ttcagacgtg tgctcttccg      60
atct                                                                   64
```

<210> 7
<211> 58
<212> DNA
<213> Artificial Sequence
<220>
<223> primer 1 for amplification after exon trapping
<400> 7
aatgatacgg cgaccaccga gatctacact ctttccctac acgacgctct tccgatct 58
<210> 8
<211> 64
<212> DNA
<213> Artificial Sequence
<220>
<223> primer 2 for amplification after exon trapping
<400> 8

```
caagcagaag acggcatacg agatcgtgat gtgactggag ttcagacgtg tgctcttccg      60
atct                                                                   64
```

## Description

## Technical Field

The present invention relates to the field of biological information, and in particular to a method, device and kit for detecting a fetal gene mutation.

## Background Art

Prenatal diagnosis, also known as intrauterine diagnosis, refers to the assessment of congenital diseases (comprising malformations and hereditary diseases) using various methods before the birth of the fetus. It provides the scientific basis for the termination of the pregnancy. Among them, prenatal diagnosis of hereditary diseases mainly targets chromosomal diseases and Mendelian inheritant disease. Mendelian inheritant disease refers to a disease transmitted according to Mendel' law, which is usually caused by a single gene mutation controlled by a pair of alleles, involving changes in a single nucleotide to the entire gene, therefore this type of disease is also called the single-gene defect. As of June 25, 2013, the OMIM (online mendelian inheritance in man) database has encompassed 4,912 single-gene defects with clear molecular mechanisms, involving 2,992 pathogenic genes.

In terms of current strategies of prenatal testing, genetic diagnosis can be divided into two main categories: direct genetic diagnosis and indirect genetic diagnosis. Direct genetic diagnosis means direct detection of the pathogenic gene itself, and such method is mainly applicable to families with clear gene mutation sites, types, and pathogenicity of probands. In terms of current tests of prenatal diagnosis for single-gene defects, genetic diagnosis can be divided into two types: pre-implantation genetic diagnosis (PGD) and prenatal diagnosis during pregnancy, depending on time periods of the diagnosis. The development of high-throughput sequencing technologies has greatly accelerated the innovation of clinical detection technologies.

Prenatal diagnosis in pregnancy comprises invasive prenatal diagnosis and non-invasive prenatal diagnosis. Non-invasive prenatal diagnosis (NIPD) is also known as a prenatal diagnosis technology that is not invasive. With the discovery of cell-free fetal DNAs (cffDNAs) in maternal plasma, non-invasive prenatal diagnosis is becoming increasingly popular due to its low risk. However, due to the very slight difference between the maternal DNA and the cell-free fetal DNA, a large amount of maternal DNA background undoubtedly increases the difficulty of detecting the cell-free fetal DNA, especially in the detection of point mutations.

AU 2015/249846 A2 describes methods, systems and, computer readable medium for detecting ploidy of chromosome segments or entire chromosomes based on phasing of the alleles and determination of individual and joint probabilities and a best-fit model.

US 2013/0123120 A1 describes methods for simultaneously amplifying multiple nucleic acid regions of interest in one reaction volume as well as methods for selecting a library of primers for use in such amplification methods.

Recently, Liao et al. and Lo et al. performed sequencing analysis on the plasma cell-free DNAs of pregnant women with a human genome coverage up to 65x. They detected over 95% of specific paternal SNPs carried by the fetus, derived genetic maps of genomes of the fetus and pregnant woman according to the sequencing results, and successfully detected a fetus with an inherited 4-bp known mutation in a thalassemia gene from the father.

Although the above-mentioned method can be used to derive the genetic map of the genome of a fetus by sequencing analysis of the plasma cell-free DNAs from a pregnant woman, it needs to combine the genetic information derived from the father. Sequencing multiple samples would undoubtedly increase the cost of sequencing significantly, and the dependence on genetic information derived from the father may also be limited. In addition, the above-mentioned method has problems of requiring whole-genome sequencing, with high sequencing depths, and only assessing mutations associated with the paternal source. Therefore, there is still a need to improve the existing detection method.

## Summary of the Invention

The present invention aims to provide a computer-implemented method and a device for detecting mutations in the fetal genome as defined in the claims, so that all SNPs of the fetus within the range of sequencing data are detected while the cost of detection is reduced.

In order to achieve the above object of the present invention, according to an aspect of the present invention, a computer-implemented method for detecting mutations in a fetal genome is provided, the method comprising the steps of:

obtaining sequencing data from high-throughput sequencing of cell-free DNA in maternal peripheral blood; aligning the sequencing data with those of a reference genome to obtain SNP sites; performing mixed genotyping for each of the SNP sites using a Bayesian model and a fetal concentration $f$ to obtain a mixed genotype with the maximum probability among seven mixed genotypes for each of the SNP sites, wherein performing mixed genotyping comprises the steps of:
calculating

$$\varphi_j = \frac{P(G_{ij} \mid S_i)}{P(G_{ij*} \mid S_i)} = \frac{P(S_i \mid G_{ij})P(G_{ij})}{P(S_i \mid G_{ij*})P(G_{ij*})} \tag{4}$$

wherein, $\varphi_j$ represents the ratio of the probability of the mixed genotype $G_j$ at the i-th SNP site to a probability of the mixed genotype $G_{j*}$ at the i-th SNP site under the $S_i$ condition,
wherein $P(S_i \mid Gij)$ is calculated as follows:

$$P(S_i \mid G_{ij}) = \binom{k+r-1}{r-1} \times f(b)^r \times (1 - f(b))^k \tag{7},$$

wherein r represents the number occurrence of a mutant allele at the i-th SNP site, k represents the number occurrence of a reference allele at the i-th SNP site,
wherein f(b) refers to the probability of the mutant allele in the mixed genotype, when the mixed genotype $G_{ij}$ is $G_{il}$, the value of the $f(b)$ is 0; when the mixed genotype $G_{ij}$ is $G_{i2}$, the value of the $f(b)$ is $f/2$; when the mixed genotype $G_{ij}$ is $G_{i3}$, the value of the $f(b)$ is 0.5-$f/2$; when the mixed genotype $G_{ij}$ is $G_{i4}$, the value of the $f(b)$ is 0.5; when the mixed genotype $G_{ij}$ is $G_{i5}$, the value of the $f(b)$ is 0.5+$f/2$; when the mixed genotype $G_{ij}$ is $G_{i6}$, the value of the $f(b)$ is 1-$f/2$; and when the mixed genotype $G_{ij}$ is $G_{i7}$, the value of the $f(b)$ is 1; wherein the $f$ represents the fetal concentration,
wherein $P(Gij)$ is calculated as follows:

$$\begin{cases} P(G' = AA) = (1-\theta)^2 \\ P(G' = AB) = 2\theta(1-\theta) \\ P(G' = BB) = \theta^2 \end{cases} \tag{6}$$

wherein $\theta$ is the population mutation frequency of the i-th SNP site, and
finding the mixed genotype with maximum occurrence probability among the seven mixed genotypes by using the formula

$$G = \arg\max(\varphi_j) \tag{5},$$

and
recording the mixed genotype with maximum occurrence probability as the mixed genotype with maximum probability at the i-th SNP site; and
step D, identifying from the fetal genotype in the mixed genotype pathogenic mutations;
wherein the mixed genotype refers to the pseudo-tetraploid genotype, which is composed of genotypes of the pregnant woman and the fetus, the mixed genotype is any one of seven types, AAAA, AAAB, ABAA, ABAB, ABBB, BBAB, and BBBB, , where A represents the reference allele of each SNP sites, and B represents the mutant allele of each SNP sites, wherein the seven types are sequentially numbered as type 1, type 2, type 3, type 4, type 5, type 6 and type 7, and
wherein the method does not include separate sequencing of paternal and/or maternal samples.

In a preferred embodiment the fetal concentration is calculated by iteration as follows:

step 0: pre-estimating that fetal concentration $f$ is a defined value which is chosen from 5 to 20%;
iteration:

step 1: inferring the fetal genotype according to the mixed genotyping as defined in claim 1, and calculating the fetal concentration $f'$ according to the $f(b)$ in the genotype;
step 2: calculating $\Delta f$, wherein $\Delta f = D(f\text{-}f')$;
step 3: if $\Delta f < \varepsilon$, the iteration ends, wherein $\varepsilon$ is less than or equal to 0.001; and
step 4: obtaining the fetal concentration $f = f$ (b);

wherein the function D( ) represents a distance function, which measures a difference between two variables; and wherein f(b) refers to the probability of the mutant allele in the mixed genotype, when the mixed genotype $G_{ij}$ is $G_{i1}$, the value *of the f(b)* is 0; when the mixed genotype $G_{ij}$ is $G_{i2}$, the value of the $f(b)$ is $f/2$; when the mixed genotype $G_{ij}$ is $G_{i3}$, the value of the $f(b)$ is 0.5-$f$/2; when the mixed genotype $G_{ij}$ is $G_{i4}$, the value of the $f(b)$ is 0.5; when the mixed genotype $G_{ij}$ is $G_{i5}$, the value of the $f(b)$ is 0.5+$f$/2; when the mixed genotype $G_{ij}$ is $G_{i6}$, the value of the $f(b)$ is 1-$f$/2; and when the mixed genotype $G_{ij}$ is $G_{i7}$, the value of the $f(b)$ is 1.

Further, the step of performing mixed genotyping for each of the SNP sites as defined above using a Bayesian model and a fetal concentration $f$ to obtain a mixed genotype with the maximum probability among seven mixed genotypes for each of the SNP sites comprises: obtaining the following formula (1) based on the sum of the conditional probability of the seven mixed genotypes is 1,

$$\sum P(G_j \mid S) = 1 \tag{1}$$

wherein $G_j$ represents any one of the seven mixed genotypes, S represents one of the SNP sites, $P(G_j \mid S)$ represents the probability of the mixed genotype $G_j$ at a SNP site under the S condition; obtaining the following formula (2) from the Bayesian model

$$P(G_{ij} \mid S_i) = \frac{P(S_i \mid G_{ij})P(G_{ij})}{P(S_i)} \tag{2}$$

wherein in the formula (2), $P(G_{ij})$ represents the probability of occurrence of $G_j$ at the i-th SNP site, and $j$ value corresponds to the sequentially numbered mixed genotype, which are 1, 2, 3, 4, 5, 6 or 7 respectively;
obtaining the following formula (3) from formula (2) by selecting any one mixed genotype $G_{j*}$ from $G_j$ as the reference mixed genotype:

$$P(G_{ij*} \mid S_i) = \frac{P(S_i \mid G_{ij*})P(G_{ij*})}{P(S_i)} \tag{3}$$

dividing each side of the formula (2) with the corespongding side of formula (3) to obtain the following formula (4)

$$\varphi_j = \frac{P(G_{ij} \mid S_i)}{P(G_{ij*} \mid S_i)} = \frac{P(S_i \mid G_{ij})P(G_{ij})}{P(S_i \mid G_{ij*})P(G_{ij*})} \tag{4}$$

wherein, $\varphi_j$ represents the ratio of the probability of the mixed genotype $G_j$ at the i-th SNP site to a probability of the mixed genotype $G_{j*}$ at the i-th SNP site under the $S_i$ condition; $P(G_{ij})$ is calculated from the population mutation frequency, and $P(S_i|G_{ij})$ is obtained by a binomial distribution formula using the number of occurrence of the mutant allele at the SNP sites, the number of occurrence of the reference allele corresponding to the mutant allele, and the initial fetal concentration $f$, then by the following formula (5)

$$G = \arg\max(\varphi_j)$$

$$(5)$$

finding the mixed genotype with maximum occurrence probability among the seven mixed genotypes, and recording the mixed genotype with maximum occurrence probability as the mixed genotype with maximum probability at the i-th SNP site.

Further, $P(G_{ij})$ in the formula (4) is obtained by multiplying the probability of genotype G' of the pregnant woman and the probability of genotype G' of the fetus, which are caculated using the following formula (6)

$$\begin{cases} P(G^{'} = AA) = (1-\theta)^2 \\ P(G^{'} = AB) = 2\theta(1-\theta) \\ P(G^{'} = BB) = \theta^2 \end{cases}$$

$$(6)$$

wherein $\theta$ is the population mutation frequency of the i-th SNP site.

Further, $P(S_i|G_{ij})$ in the formula (4) is calculated by the following formula (7):

$$P(S_i \mid G_{ij}) = \binom{k+r-1}{r-1} \times f(b)^r \times (1-f(b))^k$$

$$(7)$$

wherein r represents the number of occurrence of the mutant allele at the i-th SNP site, $k$ represents the number of occurrence of the reference allele at the i-th SNP site, and $f(b)$ represents the theoretical probability of the occurrence of a mutant allele when the mixed genotype of the i-th SNP site is $G_{ij}$.

Further, depending on the mixed genotype $G_{ij}$, the theoretical probability $f(b)$ of the occurrence of a mutant allele is respectively calculated as follows, when the mixed genotype of the i-th SNP site is $G_{ij}$: when the mixed genotype of the i-th SNP site is $G_{i1}$, the value of the $f(b)$ is 0; when the mixed genotype $G_{ij}$ is $G_{i2}$, the value of the $f(b)$ is $f$/2; when the mixed genotype $G_{ij}$ is $G_{i3}$, the value of the $f(b)$ is 0.5-$f$/2; when the mixed genotype $G_{ij}$ is $G_{i4}$, the value of the $f(b)$ is 0.5; when the mixed genotype $G_{ij}$ is $G_{i5}$, the value of the $f(b)$ is 0.5+$f$/2; when the mixed genotype $G_{ij}$ is $G_{i6}$, the value of the $f(b)$ is 1-$f$/2; and when the mixed genotype $G_{ij}$ is $G_{i7}$, the value of the $f(b)$ is 1; wherein the $f$ represents the fetal concentration.

Further, the fetal concentration may be calculated by iteration using a pre-estimated fetal concentration, preferably the pre-estimated fetal concentration is 10%; and more preferably the pre-defined value is $\leq 0.001$.

Further, the second mixed genotype is selected from any one or two or more of the following four mixed genotypes: AAAB, ABAA, ABBB, and BBAB.

Further, the step of identifying from the fetal genotype in the mixed genotype pathogenic mutations comprises: filtering the polymorphic sites with a high incidence in the human population in a fetal genotype in the target mixed genotype of each of the SNP sites to obtain preliminary candidate mutation sites; filtering SNP sites of synonymous mutations and nonsense mutations and mutations occuring in a non-conserved regions, from the preliminary candidate mutation sites to obtain candidate mutation sites; and performing a literature and database search for information associated with a monogenic disease on the candidate mutation sites to obtain the pathogenic mutations. According to another aspect of the present invention, a device for detecting gene mutations is provided, the device comprising: a detection module for performing high-throughput sequencing of cell-free DNA existed in peripheral blood of a pregnant woman to obtain sequencing data; an alignment module for aligning the sequencing data with a reference genomic sequence to obtain SNP sites; a target mixed genotype determination module for performing mixed genotyping at each SNP sites using a Bayesian model and a fetal concentration $f$ to obtain a mixed genotype with the maximum probability among seven mixed genotypes of each SNP sites as defined above, and taking the mixed genotype with the maximum probability as the target mixed genotype of each of the SNP sites; and a mutation site screening module for identifying from the fetal genotype in the mixed genotype pathogenic mutations in the target mixed genotype of each of the SNP sites; wherein the mixed genotype refers to the pseudo-tetraploid genotypes, which is composed of genotypes of the pregnant woman and her fetus, the mixed genotype is any one of seven types, AAAA, AAAB, ABAA, ABAB, ABBB, BBAB, and BBBB, where A represents a reference allele of each of the SNP sites, and B represents a mutant allele of each of the SNP sites, the seven types are sequentially numbered as type 1, type 2, type 3, type 4, type 5, type 6 and type 7.

Further, the mutation site screening module comprises: a high-incidence polymorphic site filtration sub-module for filtering out polymorphic sites with high incidence in the human population in a fetal genotype in the target mixed genotype of each of the SNP sites to obtain preliminary candidate mutation sites; a gene mutation screening sub-module for filtering SNP sites of synonymous mutations, nonsense mutations and mutations occuring in non-conserved regions, from the preliminary candidate mutation sites to obtain candidate mutation sites; and a literature and clinical data review sub-module for performing a literature and database search for information associated with a monogenic disease on the candidate mutation sites to obtain the pathogenic mutations. Applying the technical solutions of the present invention, SNP sites having mixed maternal and fetal genomic informations can be obtained by high-throughput sequencing and alignment with a reference genomic sequence, and genotypes of cell-free fetal DNA and the mother's own DNA in the peripheral blood of the pregnant woman can be typed using the pseudo-tetraploid genotyping model proposed by the present invention, thereby enabling the detection of all possible gene mutations in the fetus only using peripheral blood of the pregnant woman. The method of the present invention reduces separate sequencing for samples derived from the father and/or mother; and has not special requirement of the sequencing technology, wherein the target region sequencing can be used to obtain sequencing data, thereby reducing the cost of sequencing. Furthermore, the present method can detect fetal gene mutations at all SNP sites within the range of sequencing data, providing convenient and diversified services for prenatal diagnosis.

**Brief Description of the Drawings**

The accompanying drawings of the descriptions constitute a part of the present application, and are used for providing further understanding of the present invention, the illustrations embodiments of the present invention and thereof are intended to explain the present invention and are not intended to limit the invention. In the drawings:

Figure 1 shows a flow chart of a method for detecting gene mutations in a preferred embodiment of the present application;

Figure 2 shows an operation flowchart in Example 1 of the present application; and

Figure 3 shows a graphical results of verification using the existing mutation detection method on the gene mutation detected by the method of the present application in Example 2.

**Detailed Description of Embodiments**

It should be noted that the embodiments and the features in the embodiments in the present application may be combined with each other without conflict. The invention will be described in detail below through the drawings in conjunction with the embodiments.

Glossaries:

The population mutation frequency refers to the proportion of mutation of a gene in a particular population, for example the mutation frequency per thousand Asian people.

The pre-defined value reflects the level of detection resolution, and can be reasonably set according to the actual situation of sequencing. For example, when the sequencing depth is $\geq 1000\times$, the preferred pre-defined value is $\leq 0.001$。

Fetal concentration is the ratio of cell-free fetal DNAs in plasma of a pregnant woman to total cell-free DNAs in plasma. The fetal concentration f can be obtained by experimental methods well known to those skilled in the art, or can be preliminarily pre-estimated according to common knowledge in the art, for example, 5% to 20%.

In the present invention, the high-throughput sequencing of cell-free DNAs in the peripheral blood of pregnant women can be either whole gemome sequencing (WGS) or target region capture sequencing of the genes of interest.

In the present invention, the mixed genotype refers to a pseudo-tetraploid genotype composed of genotypes of a pregnant woman and a fetus, and both A and B are haplotypes. The first two haplotypes of the mixed genotype represent the diploid genotype of the mother, and the latter two haplotypes represent the diploid genotype of the fetus. A represents a reference allele of each of the SNP site, and B represents a mutant allele of each of the SNP site. For a site of the sequencing data, if it is consistent with the base of the corresponding site in the reference genome, it is a reference genotype, and otherwise it is a mutant genotype. The mixed genotype, for example, may be AAAB, which means that the diploid genotype of the mother is AA and is a homozygous reference type, and the diploid genotype of the fetus is AB and is a mutantion carring type.

In the present invention, the population mutation frequency refers to the proportion of the number of cells or individuals in which a mutation occurs within a specific population, for example the mutation frequency per thousand Asians.

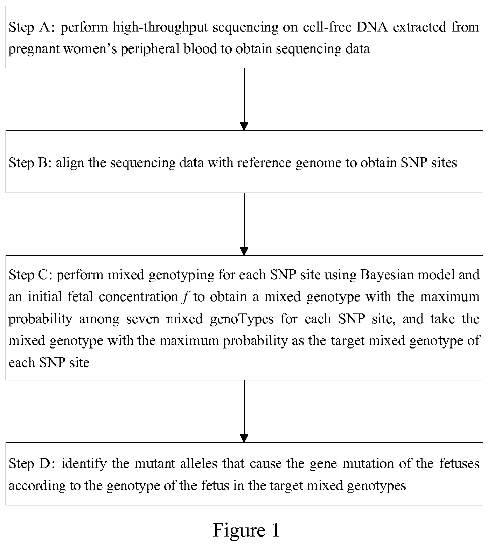

As mentioned in the background art section, the method for detecting fetal gene mutations using high-throughput sequencing in the prior art typically requires additional paternal and maternal sample information and can only detect a Y chromosome-linked monogenic disease. In order to reduce the detection cost and provide diversified prenatal testing services, a computer-implemented method for detecting mutations in a fetal genome is provided in a typical embodiment of the present invention, as shown in Figure 1, the method comprising the steps as defined above of: performing high-throughput sequencing of cell-free DNAs in peripheral blood of a pregnant woman to obtain sequencing data; aligning the sequencing data with a reference genome to obtain SNP sites; performing mixed genotyping for each of the SNP sites using a Bayesian model and a fetal concentration $f$ to obtain a mixed genotype with the maximum probability among seven mixed genotypes for each of the SNP sites, and taking the mixed genotype with the maximum probability as a target mixed genotype of each of the SNP sites; and identifying from the fetal genotype in the mixed genotype pathogenic mutations in the target mixed genotype; wherein the mixed genotype refers to pseudo-tetraploid genotypes formed by genotypes of the pregnant woman and the fetus, the mixed genotype is any one of seven types consisting of AAAA, AAAB, ABAA, ABAB, ABBB, BBAB, and BBBB are sequentially numbered as type 1, type 2, type 3, type 4, type 5, type 6 and type 7, where A represents a reference allele of each of the SNP sites, and B represents a mutant allele of each of the SNP sites.

Based on the fact that cell-free DNA in the peripheral blood of the pregnant woman comprises both the maternal DNA and the fetal DNA, and current technical means are difficult to completely separate the DNA from two sources, the concept of pseudo-tetraploid is proposed by the inventor, the tetraploid obtained by mixing genotypes of the pregnant woman and the fetus is called pseudo-tetraploid, and at each site of the genome, the genotype of the site obtained by mixing the genotype of the pregnant woman and the genotype of the fetus is called a mixed genotype. In order to assess the probability of occurance of a mutant genotype at each site, A represents the normal reference allele at that site; B represents the mutant allele at the site.

By placing the diploid genotype at each site of the pregnant woman in front, and placing the diploid genotype at the corresponding site of the fetus behind to indicate a mixed genotype at a site of the pseudo-tetraploid, seven possible mixed genotypes of AAAA, AAAB, ABAA, ABAB, ABBB, BBAB and BBBB can be obtained, a mixed genotype with the maximum probability at each of the SNP sites can be deduced from sequencing data to obtain a target mixed genotype at the site, thereby obtaining the genotype of the fetus from the target mixed genotype.

Further, the inventors proposed the idea of mixed genotyping of the above-mentioned pseudo-tetraploid using conditional probability and the Bayesian model.

By the above-mentioned method of the present invention, SNP sites having mixed maternal and fetal genomic informations can be obtained only by performing high-throughput sequencing and sequence alignment of cell-free DNA in peripheral blood of the mother; the fetal and the maternal genotype at each of the SNP sites of cell-free DNA in the peripheral blood of the pregnant woman can be determined based on the concept of mixed genotyping proposed by the present invention, thereby achieving detection of all possible gene mutations in the fetus using only peripheral blood of the pregnant woman. On one hand, the present method reduces the sequencing of the paternal and maternal samples, and reduces the cost of sequencing; and on the other hand, it also facilitates the detection of fetal gene mutations under certain special conditions, such as the case where the paternal sample is not available, and thus provides diversified services for prenatal diagnosis.

In the above-mentioned method of the present invention, based on the concepts of pseudo-tetraploid and mixed genotyping of pseudo-tetraploid proposed by the present invention, those skilled in the art can perform genotyping for a mixed genotype of pseudo-tetraploid using the conditional probability and a Bayesian model, so as to obtain the genotype of the fetus at the SNP site, which lays a foundation for screening mutation sites that cause fetal gene mutations. According to the sources of peripheral blood samples of pregnant women, an initial fetal concentration is divided into two cases: known or unknown. When the fetal concentration is known, the initial fetal concentration $f$ is a true fetal concentration. A mixed genotype with the maximum probability for each of the SNP sites can be calculated using the initial fetal concentration $f$ and the Bayesian model. When the fetal concentration is unknown, a derivation process for the true fetal concentration is required.

In a preferred embodiment of the present invention, the fetal concentration is calculated by iteration as follows:

step 0: pre-estimating that fetal concentration $f$ is a defined value which is chosen from 5 to 20%;
iteration:

step 1: inferring the fetal genotype according to the mixed genotyping as defined in claim 1, and calculating the fetal concentration $f'$ according to the $f(b)$ in the genotype;
step 2: calculating $\Delta f$, wherein $\Delta f = D(f-f')$;

step 3: if $\Delta f < \varepsilon$, the iteration ends, wherein $\varepsilon$ is less than or equal to 0.001; and

step 4: obtaining the fetal concentration $f = f$ (b);

wherein the function D( ) represents a distance function, which measures a difference between two variables; and wherein f(b) refers to the probability of the mutant allele in the mixed genotype, when the mixed genotype $G_{ij}$ is $G_{i1}$, the value *of the f(b)* is 0; when the mixed genotype $G_{ij}$ is $G_{i2}$, the value of the $f(b)$ is $f/2$; when the mixed genotype $G_{ij}$ is $G_{i3}$, the value of the $f(b)$ is 0.5-$f/2$; when the mixed genotype $G_{ij}$ is $G_{i4}$, the value of the $f(b)$ is 0.5; when the mixed genotype $G_{ij}$ is $G_{i5}$, the value of the $f(b)$ is 0.5+$f/2$; when the mixed genotype $G_{ij}$ is $G_{i6}$, the value of the $f(b)$ is 1-$f/2$; and when the mixed genotype $G_{ij}$ is $G_{i7}$, the value of the $f(b)$ is 1.

In the above-mentioned process for mixed genotyping of pseudotetraploid, since the fetal concentration is unknown, the occurrence probability of seven possible mixed genotypes is calculated at a fetal concentration that is preliminarily pre-estimated according to common sense, for example, 5% to 15%, as the initial fetal concentration, thereby obtaining the mixed genotype with the maximum probability at each of the SNP sites. In combination with the actual sequencing data, the actual fetal concentration is calculated by using the mutations from the mother or the fetus, and then the calculated fetal concentration is then compared with the pre-estimated initial fetal concentration. If the difference is less than a pre-defined value, then the calculated fetal concentration needs to be taken as the concentration in step 0, and the steps 1 to 4 are repeated until the difference between the calculated fetal concentration at some point and the initial concentration in step 0 of the cycle is less than the pre-defined value, After the termination of the cycle, the mixed genotype with the maximum probability at each of the SNP sites at the initial concentration in the step 0 of the cycle is recorded as the target mixed genotype of each of the SNP sites. The above-mentioned pre-defined value reflects the level of the detection resolution and can be reasonably set according to the actual situation. For example, when the sequencing depth is $\geq 1000 \times$, a preferred pre-defined value is $\leq 0.001$.

In the above-mentioned preferred embodiment, the selection principle of the mixed genotype for facilitating calculation of the fetal concentration can be rationally selected according to the calculation method. In a preferred embodiment of the invention, the above-mentioned mixed genotype for calculation of the fetal concentration includes, but not limited to, any one or more of AAAB, ABAA, ABBB, and BBAB. The mutant alleles or reference alleles in these mixed genotypes are only from the pregnant woman or the fetus, and the concentration of one of them can be calculated based on the number of times the mutant alleles and the reference alleles are detected in the sequencing data, so that it is very easy to obtain the concentration in the fetus.

According to the present invention, the above-mentioned step of performing mixed genotyping for each of the SNP sites using a Bayesian model and a fetal concentration $f$ to obtain a mixed genotype with the maximum probability among seven mixed genotypes for each of the SNP sites as defined above comprises: obtaining the following formula (1) based on the sum of the conditional probability of the seven mixed genotypes is 1, wherein $G_j$ represents any one of the seven mixed genotypes, S represents one of the SNP sites,

$$\sum P(G_j \mid S) = 1 \qquad (1)$$

and $P(G_j|S)$ represents the probability of the mixed genotype of the SNP site being $G_j$ at an SNP site under the S conditong; obtaining the following formula (2) from the Bayesian model

$$P(G_{ij} \mid S_i) = \frac{P(S_i \mid G_{ij})P(G_{ij})}{P(S_i)} \qquad (2)$$

wherein in the formula (2), $P(G_{ij})$ represents the occurrence probability of $G_j$ genotype at the i-th SNP site, and $j$ value corresponds to the sequentially numbered mixed genotype, which are 1, 2, 3, 4, 5, 6 or 7 respectively; and obtaining the following formula (3) from the formula (2) by selecting any one mixed genotype $G_{j*}$ from $G_j$ as the reference mixed genotype:

$$P(G_{ij*} \mid S_i) = \frac{P(S_i \mid G_{ij*})P(G_{ij*})}{P(S_i)}$$

$$(3)$$

dividing each side of the formula (2) with the corresponding side of formula (3) to obtain the following formula (4)

$$\varphi_j = \frac{P(G_{ij} \mid S_i)}{P(G_{ij*} \mid S_i)} = \frac{P(S_i \mid G_{ij})P(G_{ij})}{P(S_i \mid G_{ij*})P(G_{ij*})}$$

$$(4)$$

wherein, $\varphi_j$ represents the ratio of the probability of the mixed genotype $G_j$ at the i-th SNP site to a probability of the mixed genotype $G_j*$ at the i-th SNP site under the $S_i$ condition; $P(G_{ij})$ is calculated from the population mutation frequency, and $P(S_i|G_{ij})$ is obtained by a binomial distribution formula using the number occurrence of a mutant allele at each of the SNP sites, the number occurrence of a reference allele corresponding to the mutant allele, and the initial fetal concentration $f$; then by the following formula (5)

$$G = \arg\max(\varphi_j)$$

$$(5)$$

finding the mixed genotype with the maximum occurrence probability among the seven mixed genotypes, and recording the mixed genotype with maximum occurrence probability as the mixed genotype with maximum probability at the i-th SNP site.

In the above-mentioned preferred embodiment of the present invention, the method for calculation of the mixed genotype with the maximum probability at a SNP site is converted into a calculation of a ratio between an occurrence probability of the above-mentioned seven mixed genotypes at the SNP site and an occurrence probability of one of the mixed genotypes, so that the mixed genotype with the maximum occurrence probability at the SNP site is indirectly obtained, and thus is inferred to be an initial mixed genotype of the site.

In the above-mentioned method of the present invention, in the case that a mutant genotype is known to occur at a site, those skilled in the art can calculate $P(G_{ij})$ in the formula (4) using the population mutation frequency in the thousand human genome database. In a preferred embodiment of the present invention, $P(G_{ij})$ is calculated by the following formula (6)

$$\begin{cases} P(G' = AA) = (1-\theta)^2 \\ P(G' = AB) = 2\theta(1-\theta) \\ P(G' = BB) = \theta^2 \end{cases}$$

$$(6).$$

In the above-mentioned formula (6), G' represents the above-mentioned three separate possible genotypes occurring at a site in the pregnant woman or the fetus, and then an occurrence probability of a mixed genotype at the specific site is the product of the occurrence probability of genotype G' at the site of the pregnant woman and the occurrence probability of genotype G' at the site of the fetus, wherein $\theta$ is the population mutation frequency of the i-th SNP site. The parameter $\theta$ is obtained from the population mutation frequency in the thousand human genome database.

In the above-mentioned method of the present invention, the numerical value of $P(S_i|G_{ij})$ is obtained depending on the difference between the number occurrence of mutant alleles and the number occurrence of reference alleles in actual sequencing data, and the difference in the initial fetal concentration when a site is a specific mixed genotype $G_{ij}$, using a binomial distribution formula. In a specific embodiment of the present invention, $P(S_i|G_{ij})$ in the formula (4) is calculated by the following formula (7):

$$P(S_i \mid G_{ij}) = \binom{k+r-1}{r-1} \times f(b)^r \times (1-f(b))^k$$

wherein r represents the number occurrence of the mutant allele at the i-th SNP site, k represents the number occurrence of the reference allele at the i-th SNP site, and $f(b)$ represents the theoretical probability of the occurrence of the mutant allele of the fetus when the mixed genotype of the i-th SNP site is $G_{ij}$.

In the above-mentioned embodiment, $f(b)$ is related to the concentration of cell-free fetal DNA in peripheral blood of the pregnant woman, and can be calculated using a conventional fetal concentration calculation method such as Fetal Quant (see Lench N, Barrett A, Fielding S, et al. The clinical implementation of non-invasive prenatal diagnosis for single-gene disorders: challenges and progress made [J]. Prenatal diagnosis, 2013, 33(6): 555-562.).

After obtaining the fetal concentration, when a site is one of the mixed genotypes, a possible paternal genotype is derived for the mixed genotype, thereby deducing the theoretical occurrence probability of a mutant allele when a specific mixed genotype occurs.

As described above, in the present invention, when the difference value between the final second fetal concentration calculated from the initial fetal concentration by iteration using the expectation maximization algorithm and the initial fetal concentration is not significantly different from the pre-defined value, the initial fetal concentration or the second fetal concentration in this case is the true concentration of the fetus in the sample. Assuming that the initial fetal concentration (pre-estimated fetal concentration) $f$ is 10%, the mixed genotypes of all SNP sites when $f$ =10% are calculated; the fetal concentration $f'$ (second fetal concentration $f'$) is calculated according to frequencies of the reference allele and the mutant allele actually detected for a mixed genotype; and if the difference value between $f$ and $f'$ is less than the pre-defined value, then the iteration ends, and the corresponding $f'$ at the end of the iteration is the true fetal concentration. More preferably, the above-mentioned pre-defined value is less than or equal to 0.001.

Specifically, the following algorithm is used to calculate the true fetal concentration:

step 0: pre-estimating that fetal concentration $f$ is 10%;
iteration:

step 1: inferring the fetal genotype according to the mixed genotyping, and calculating the fetal concentration $f'$ according to the $f(b)$ in the genotype;
step 2: calculating $\Delta f$, wherein $\Delta f$ = D($f$-$f'$);
step 3: if $\Delta f < \varepsilon$, the iteration ends, wherein $\varepsilon$ represents any small positive number; and
step 4: obtaining the fetal concentration $f$ = $f$ (b);

wherein the function D( ) represents a distance function, which measures a difference between two variables.

The process of calculation of the true fetal concentration herein is illustrated below by examples.

For example, 3 SNP sites are selected for calculation, all the genotypes are assumed as AAAB type, $f$ is the initial pre-estimated fetal concentration, and $f'$ is the second fetal concentration deduced from the detected frequencies of A and B.

For the first SNP, A and B are detected 19 times and once respectively, assuming $f$=10%, the probability values of the 7 genotypes are calculated, and it is derived that the mixed genotype of the SNP is AAAA after comparison, which does not meet the hypothesis of AAAB, and the first SNP should be removed;

for the second SNP, A and B are detected 16 times and 4 times respectively, assuming $f$ =10%, the probability values of the 7 genotypes are calculated, and it is derived that the mixed genotype of the SNP is AAAB after comparison, which meets the hypothesis of AAAB, and in this case, $f'$=40%; and

for the third SNP, A and B are detected 18 times and twice respectively, assuming $f$ =10%, the probability values of the 7 genotypes are calculated, and it is derived that the mixed genotype of the SNP is AAAB after comparison, which meets the hypothesis of AAAB, and in this case, $f'$=20%.

Combining cases of the above three SNPs, the second and the third cases are accepted, the first one is excluded, then the average value of $f'$ is 30%, and the difference value between the assumed $f$ and the detected and deduced $f'$ is greater than 0.001; therefore, iterative calculation needs to continue until the difference value there between is less than 0.001.

The above-mentioned method for calculating the fetal concentration $f$ by the iterative algorithm of the present invention has advantages of high accuracy and no limitation by the gender, as compared with the method for calculating the fetal concentration *f* using X chromosome in a male fetus and a methylation method in a female fetus in the prior art.

After obtaining the fetal concentration using the above-mentioned method, depending on the mixed genotype $G_{ij}$ the theoretical probability *f(b)* of the occurrence of the mutant allele can be respectively calculated according to the following formulas, when a mixed genotype of the i-th SNP site is $G_{ij}$: when the mixed genotype $G_{ij}$ is $G_{i1}$, the value of the *f(b)* is 0; when the mixed genotype $G_{ij}$ is $G_{i2}$, the value of the *f(b)* is *f*/2; when the mixed genotype $G_{ij}$ is $G_{i3}$, the value of the *f(b)* is 0.5-*f*/2; when the mixed genotype $G_{ij}$ is $G_{i4}$, the value of the *f(b)* is 0.5; when the mixed genotype $G_{ij}$ is $G_{i5}$, the value of the *f(b)* is 0.5+*f*/2; when the mixed genotype $G_{ij}$ is $G_{i6}$, the value of the *f(b)* is 1-*f*/2; and when the mixed genotype $G_{ij}$ is $G_{i7}$, the value of the *f(b)* is 1; wherein the *f* represents the initial fetal concentration.

*f(b)* refers to the probability of the mutant allele in the mixed genotype of pseudo-tetraploid, and thus only the occurrence of B in the mixed genotype of pseudo-tetraploid needs to be calculated, as shown in the Table 1 below: (assuming that the probability of mixed genotypes of pseudo-tetraploid is 1)

Table 1:

| Mixed genotyp e | Mother | | Fetus | | Occurrence probability f(b) of B genotype |
|---|---|---|---|---|---|
| | Genotyp e | Concentrati on | Genotyp e | Concentrati on | |
| 1-AAA A | AA | 1-*f* | AA | *f* | B does not occur, so it is 0 |
| 2-AAA B | AA | 1-*f* | AB | *f* | *f*/2 |
| 3-ABA A | AB | 1-*f* | AA | *f* | (1-*f*)/2=0. 5-*f*/2 |
| 4-ABA B | AB | 1-*f* | AB | *f* | 1/2 |
| 5-ABBB | AB | 1-*f* | BB | *f* | (1-*f*)/2+*f*=0. 5+*f*/2 |
| 6-BBAB | BB | 1-*f* | AB | *f* | 1-*f*/2 |
| 7-BBBB | BB | 1-*f* | BB | *f* | 1 |

In the method of the present invention, the above-described mixed genotyping enables the deducing of the target mixed genotype of each SNP sites, thereby obtaining the genotype of the fetus. After obtaining the genotype of the fetus, the pathogenic mutations leading to a gene mutation can be found. In a particular embodiment of the present invention, the step of identifying the mutation from SNP sites according to a difference in the fetal genotype in the target mixed genotype of each of the SNP sites comprises: filtering the polymorphic sites with a high incidence in the human population in various SNP sites for which fetal genotypes are deduced, to obtain preliminary candidate mutation sites; filtering SNP sites of synonymous mutations, nonsense mutations and mutations occuring in non-conserved regions, from the preliminary candidate sites to obtain candidate mutation sites; and performing literature review and clinical data review on the candidate mutation sites to obtain the mutations leading to the fetal gene mutation.

In the above-mentioned embodiment, in the process of analyzing each SNP site of the fetus to find the pathogenic mutations, the high-frequency SNP sites which cause differences between different individuals in the human population are deleted, because these sites are obviously not the pathogenic mutations. In the present invention, the high-incidence polymorphic sites in the human population are removed using the dnSNP135 public database and the Freq_1000g2012feb (thousand human genome) database which have been collated by the medical community. After removing the SNP sites caused by individual differences, preliminary candidate mutationes are obtained, and then mutation prediction softwares commonly used in the field is used to filter harmful mutations, for example, ANNOVAR software can screen whether the mutations cause amino acid change, that is, whether the mutations cause asense mutations, and can also filter whether the mutations occur in conserved sequence regions.

After the above-mentioned software filtering, it is also necessary to perform artificial interpretation of possible pathogenic mutations that have been identified. The so-called "artificial interpretation" means to find the site information associated with a monogenic disease from possible pathogenic mutations by the review of existing databases and literatures, and perform corresponding interpretation. Furthermore, the method of the present invention is not limited to detecting the presence or absence of hot spot mutations leading to known monogenic diseases, it can also detecting non-hot spot mutations of known monogenic diseases and unreported potential pathogenic genes and their mutations. Therefore, the method can provide diversified services to customers according to their different needs.

In the above-mentioned methods of the present invention, the step of performing high-throughput sequencing of cell-free DNA in peripheral blood of a pregnant woman to obtain sequencing data comprises performing sample DNA library construction at first using the high-throughput sequencing method commonly used in the art, and then sequencing using existing high-throughput sequencing platforms. In a preferred embodiment of the present invention, the step of performing high-throughput sequencing of cell-free DNA in peripheral blood of a pregnant woman to obtain sequencing data comprise: extracting plasma DNA from the peripheral blood of the pregnant woman, and enriching cell-free DNA in the plasma DNA to obtain enriched DNA; performing library construction for the enriched DNA to obtain a sequencing library; and performing high-throughput sequencing of the sequencing library to obtain the sequencing data.

In the above-mentioned preferred embodiment, since the amount of the cell-free DNA in the peripheral blood of the pregnant woman is relatively low in the maternal plasma, which is substantially not more than 10%, the step of extraction and enrichment of the cell-free DNA is required before the high-throughput sequencing. For the extraction and enrichment step, suitable extraction and enrichment methods can be selected by those skilled in the art depending on the diversity of samples and the requirement of data respectively. For example, QIAmp DNA Blood Mini Kit from Qiagen, Germany, or commercially available similar reagents from other companies, or self-made relevant reagents for extraction and enrichment of peripheral blood of a pregnant woman can be used for the extraction and enrichment.

After the step of performing library construction for the above-mentioned enriched DNA to obtain a sequencing library, different target regions are selected for sequencing, depending on detection purposes of different samples. During the actual operation of the present invention, the step of performing target region sequencing on the library to obtain a sequencing library containing the target regions is also included before performing the high-throughput sequencing. In a more preferred embodiment of the invention, the step of performing exon capture hybridization on the sequencing library is added, so that the subsequent high throughput sequencing is performed only for exons. Since introns are usually cleaved off during the transcription process of a gene, and exons are final regions encoding a protein, only performing exon sequencing can increase an effective data volume and improve efficiency of sequencing. After obtaining a sequencing library for exons, it is also possible to detect mutations of known monogenic diseases within specific target regions depending on detection purposes and/or detection samples, or to detect all mutations in the sequencing data as entirety.

In the above-mentioned preferred embodiment of the invention, different methods or reagents may be selected for capture depending on the target regions to be captured. For example, a capture kit from Roche NimbleGen, US, or a self-made kit or a commercially available kit from other companies with a similar function can be used to perform target region sequencing.

In another typical implementation of the present invention, a device for detecting gene mutations is provided, the device comprising: a detection module for performing high-throughput sequencing of cell-free DNA existed in peripheral blood of a pregnant woman to obtain sequencing data; an alignment module for aligning the sequencing data with a reference genomic sequence to obtain SNP sites; a target mixed genotype determination module for performing mixed genotyping at each SNP site using a Bayesian model and a fetal concentration $f$ to obtain a mixed genotype with the maximum probability among seven mixed genotypes of each SNP site as defined above, and taking the mixed genotype with the maximum probability as the target mixed genotype of each of the SNP sites; and a mutation site screening module for identifying from the fetal genotype in the mixed genotype pathogenic mutations in the target mixed genotype of each of the SNP sites; wherein the mixed genotype refers to the pseudo-tetraploid genotypes, which is composed of genotypes of the pregnant woman and the fetus, and the mixed genotype is any one of seven types, AAAA, AAAB, ABAA, ABAB, ABBB, BBAB, and BBBB, where A represents a reference allele of each of the SNP sites, and B represents a mutant allele of each of the SNP sites, the seven types are sequentially numbered as type 1, type 2, type 3, type 4, type 5, type 6 and type 7.

In the above-mentioned device of the present invention, SNP sites in the maternal and fetal genomic information different from those of a reference genome are obtained by the detection module and the alignment module, and a mixed genotype of the pseudo-tetraploid genotypes composed of genotypes of the pregnant woman and the fetus is typed by the target mixed genotype determination module to obtain genotypes of the mother and the fetus at each of the SNP sites, thereby achieving detection of all possible gene mutations in the fetus using only the peripheral blood sample of the pregnant woman. The device of the present invention not only reduces the separate sequencing of the paternal and maternal samples (cellular genome samples from the peripheral blood), and reduces the cost of sequencing, but also facilitates the detection of fetal gene mutations under certain special conditions, such as the case where the paternal sample is not available, and thus provides diversified services for prenatal diagnosis.

In the above-mentioned target mixed genotype determination module of the present invention, when the fetal concentration is the true fetal concentration, those skilled in the art would obtain a target mixed genotype of each of the SNP sites by modifying the conditional probability and the Bayesian model, based on the concepts of pseudo-tetraploid and the mixed genotype of pseudo-tetraploid proposed by the present invention. The above-mentioned specific calculation method for calculating a mixed genotype with the maximum probability among 7 mixed genotypes for each of the SNP sites using the initial fetal concentration $f$ can be obtained by modifying the conditional probability and the Bayesian model in many ways. Preferably, when calculating the probabilities of the seven mixed genotypes, probability calculation formulas of the seven mixed genotypes are divided by a probability calculation formula of one specific mixed genotype respectively, thereby obtaining a ratio between the probability of each mixed genotype and the probability of the specific mixed genotype, a mixed genotype with the largest ratio is the mixed genotype with the maximum probability at the SNP site, i.e. the initial mixed genotype of the SNP site. The above-mentioned specific mixed genotype may be any one of seven mixed genotypes, and may be reasonably selected according to convenience of calculation.

In the calculation module in the above-mentioned preferred embodiment, the selection principle of the mixed genotype for calculation of the fetal concentration can be rationally selected according to the calculation method. In a preferred embodiment of the invention, the above-mentioned mixed genotype for calculation of the fetal concentration includes, but is not limited to, any one or more of AAAB, ABAA, ABBB, and BBAB. The mutant alleles or reference alleles in these mixed genotypes are only from the pregnant woman or the fetus, and the concentration of one of them can be calculated based on the number occurance of the mutant alleles and the reference alleles are detected in the sequencing data, so that it is very easy to obtain the concentration of the fetus.

According to the present invention, the step of performing mixed genotyping at each SNP site by the target mixed genotype determination module using a Bayesian model and a fetal concentration $f$ to obtain a mixed genotype with the maximum probability among seven mixed genotypes of each of the SNP sites as defined above comprises: obtaining the following formula (1) based on the sum of the conditional probability of the seven mixed genotypes is 1, wherein $G_j$ represents any one of the seven mixed genotypes, S represents one of the SNP sites,

$$\sum P(G_j \mid S) = 1 \tag{1}$$

and $P(G_j \mid S)$ represents the probability of the mixed genotype $G_j$ at a SNP site under the S condition; obtaining the following formula (2) from the Bayesian model

$$P(G_{ij} \mid S_i) = \frac{P(S_i \mid G_{ij})P(G_{ij})}{P(S_i)} \tag{2}$$

wherein in the formula (2), $P(G_{ij})$ represents the probability of occurrence of $G_j$ at the i-th SNP site, and $j$ value corresponds to the sequentially numbered mixed genotype, which are 1, 2, 3, 4, 5, 6 or 7 respectively;

obtaining the following formula (3) from formula (2) by selecting any mixed genotype $G_{j^*}$ from $G_j$ as the reference mixed genotype::

$$P(G_{ij^*} \mid S_i) = \frac{P(S_i \mid G_{ij^*})P(G_{ij^*})}{P(S_i)} \tag{3}$$

dividing each side of the formula (2) with the corresponding side of formula (3) to obtain the following formula (4)

$$\varphi_j = \frac{P(G_{ij} \mid S_i)}{P(G_{ij^*} \mid S_i)} = \frac{P(S_i \mid G_{ij})P(G_{ij})}{P(S_i \mid G_{ij^*})P(G_{ij^*})} \tag{4}$$

wherein, $\varphi_j$ represents the ratio of the probability of the mixed genotype $G_j$ at the i-th SNP site to a probability of the mixed genotype $G_{j^*}$ at the i-th SNP site under the $S_i$ condition; $P(G_{ij})$ is calculated from the population mutation frequency, and $P(S_i|G_{ij})$ is obtained by a binomial distribution formula using the number occurrence of the mutant allele at each the SNP site, the number occurrence of the reference allele corresponding to the mutant allele, and initial fetal concentration $f$; then by the following formula (5)

$$G = \arg\max(\varphi_j) \tag{5}$$

finding the mixed genotype with the maximum occurrence probability among the seven mixed genotypes, and recording the mixed genotype with the maximum occurrence probability as the initial mixed genotype with the maximum probability at the i-th SNP site.

In the above-mentioned preferred embodiment of the present invention, the method for calculation of the mixed genotype at a SNP site using the conditional probability and the Bayesian model is converted into a calculation of a ratio between an occurrence probability of the above-mentioned seven mixed genotypes at the SNP site and an occurrence probability of one of the mixed genotypes, so that the mixed genotype with the maximum occurrence probability at each of the SNP sites is indirectly obtained, and thus is inferred to be the mixed genotype with the maximum probability at each of the SNP sites.

In the above-mentioned device of the present invention, in the case that a mutant genotype is known to occur at a site, those skilled in the art can calculate $P(G_{ij})$ in the formula (4) using the population mutation frequency in the thousand human genome database. In a preferred embodiment of the present invention, in the above-mentioned fetal genotype determination module (a target mixed genotype determination module), $P(G_{ij})$ in the formula (4) is calculated by the following formula (6)

$$\begin{cases} P(G^{'} = AA) = (1-\theta)^2 \\ P(G^{'} = AB) = 2\theta(1-\theta) \\ P(G^{'} = BB) = \theta^2 \end{cases}$$

$$(6).$$

In the above-mentioned formula (6), G' represents the above-mentioned three separate possible genotypes occurring at each site in the pregnant woman or the fetus, and then an occurrence probability of a mixed genotype at the specific site is the product of the probability of genotype G' at the site of the pregnant woman and the probability of genotype G' at the site of the fetus, wherein $\theta$ is the population mutation frequency of the i-th SNP site. The parameter $\theta$ is obtained from the population mutation frequency in the thousand human genome database.

In the above-mentioned devices of the present invention, in the fetal genotype determination module, the numerical value of $P(S_i|G_{ij})$ in the formula (4) is obtained depending on the difference between the number occurrence of mutant alleles and the number occurrence of reference alleles in actual sequencing data, and the difference in the initial fetal concentration when a site is a specific mixed genotype $G_{ij}$, using a binomial distribution formula. In a specific embodiment of the present invention, $P(S_i|G_{ij})$ in the formula (4) is calculated by the following formula (7):

$$P(S_i | G_{ij}) = \binom{k+r-1}{r-1} \times f(b)^r \times (1-f(b))^k$$

$$(7)$$

wherein r represents the number occurrence of the allele at the i-th SNP site, k represents the number occurrence of the reference allele at the i-th SNP site, and f(b) represents the theoretical probability of the occurrence of a mutant allele in the fetus when the mixed genotype of the i-th SNP site is $G_{ij}$.

In the above-mentioned embodiment, *f(b)* is related to the concentration of cell-free fetal DNA in peripheral blood of the pregnant woman, and can be calculated using a conventional fetal concentration calculation method such as Fetal Quant (see Lench N, Barrett A, Fielding S, et al. The clinical implementation of non-invasive prenatal diagnosis for single-gene disorders: challenges and progress made [J]. Prenatal diagnosis, 2013, 33(6): 555-562.).

After obtaining the fetal concentration, when a site is one of the mixed genotypes, a possible paternal genotype is derived for the mixed genotype, thereby deducing the theoretical occurrence probability of a mutant allele in the fetus when a specific mixed genotype occurs.

In another preferred embodiment of the present invention, the initial fetal concentration *f* is calculated by iteration using the expectation maximization algorithm. Assuming that the initial pre-estimated fetal concentration *f* is 10%, the mixed genotypes of all SNP sites when *f* =10% are calculated; the actual fetal concentration *f'* is calculated according to frequencies of the reference genotypes and the mutant alleles actually detected for some mixed genotypes; and if the difference value between *f* and *f'* is less than the pre-defined value, then the iteration ends, and the corresponding *f'* at the end of the iteration is the fetal concentration *f*. More preferably, when the above-mentioned pre-defined value is less than or equal to 0.001, the iteration ends. The specific algorithm used is the same as that in the foregoing detection method, and details are not described herein again. Similarly, when the above-mentioned mixed genotype $G_{ij}$ of the i-th SNP site is any one of the 7 types, the theoretical probability *f(b)* of the occurrence of the mutant allele is the same as in Table 1.

In the above-mentioned device of the present invention, in the above-mentioned target mixed genotype determination module, a target mixed genotype can be deduced by the pseudo-tetraploid genotyping for each of the SNP sites, thereby a fetal genotype at each of the SNP sites can be obtained from the target mixed genotype, and pathogenic mutations can thus be found after finding the fetal genotype. In a typical embodiment of the present invention, the mutation site screening module in the above-mentioned device comprises: a high-incidence polymorphic site filtration sub-module for filtering out polymorphic sites with high incidence in the human population in a fetal genotype in the target mixed genotype of each of the SNP sites to obtain preliminary mutation sites; a gene mutation site screening sub-module for filtering SNP sites of synonymous mutations, nonsense mutations and mutations occuring in non-conserved regions, from the preliminary mutation sites to obtain candidate mutation sites; and a literature and clinical data review sub-module for performing screening on the candidate mutation sites to obtain the mutations leading to pathogenic gene mutations which has been recorded in literatures and clinical data.

In the above-mentioned embodiment, in the process of analyzing each SNP site of the fetus to find the pathogenic mutations, the high-frequency SNP sites which cause individual differences in the human population are deleted by the high-incidence polymorphic site filtration sub-module, because these sites are obviously not the pathogenic mutations. In the present invention, the high-incidence polymorphic sites in the human population are removed by the above-mentioned high-incidence polymorphic site filtration sub-module using the dnSNP135 public database and the Freq_1000g2012feb (thousand human genome) database which have been collated by the medical community. After removing the SNP sites caused by individual differences, fetus-specific SNP sites are obtained, and then the sites which actually cause a gene mutation is screened by the gene mutation site screening sub-module. The module can use a mutation prediction module commonly used in the art for filtering harmful mutations. ANNOVAR module can screen whether the mutations cause amino acid change, that is, whether the mutations cause sense mutations, and can also filter whether the mutations occur in conserved sequence regions.

After the above-mentioned gene mutation site screening sub-module is subjected to the above-mentioned mutation prediction module screening, an artificial interpretation sub-module for artificial interpretation of possible pathogenic mutations that have been filtered is further included. The so-called "artificial interpretation sub-module" means to perform alignment between SNP sites which have been filtered by the mutation prediction module and pathogenic sites which are reviewed from existing databases and literatures, so as to find the site information associated with monogenic diseases and perform corresponding interpretation. The above-mentioned device of the present invention is not limited to detecting the presence or absence of hot spot mutation site leading to a known monogenic disease, it can also detect non-hot spot mutations of known monogenic diseases and unreported potential pathogenic genes and their mutations. Therefore, the method can provide diversified services to customers according to their different needs.

In the above-mentioned devices of the present invention, the detection module is a process for preparing a sequencing library from cell-free DNA enriched from peripheral blood plasma of a pregnant woman and performing high-throughput sequencing to obtain sequencing data. The step of high-throughput sequencing of cell-free DNA in peripheral blood of a pregnant woman to obtain sequencing data comprises performing sample DNA library construction at first by the high-throughput sequencing method commonly used in the art, and then sequencing using existing high-throughput sequencing platforms. In a preferred embodiment of the present invention, the above-mentioned detection device comprises a process of extracting plasma DNA from the peripheral blood of the pregnant woman, and enriching cell-free DNA in the plasma DNA to obtain enriched DNA; performing library construction for the enriched DNA to obtain a sequencing library; and performing high-throughput sequencing of the sequencing library to obtain the sequencing data.

In the above-mentioned preferred embodiment, since the amount of the cell-free DNA in the peripheral blood of the pregnant woman is relatively low in the maternal plasma, which is substantially not more than 10%, the above-mentioned detection device further comprises the step of extraction and enrichment of the cell-free DNA before the high-throughput sequencing. Suitable extraction and enrichment methods can be selected depending on diversity samples and requirement data respectively. For example, QIAmp DNA Blood Mini Kit from Qiagen, Germany, or commercially available similar reagents from other companies, or self-made relevant reagents for extraction and enrichment of peripheral blood of a pregnant woman can be used for the extraction and enrichment.

In the above-mentioned detection device, after the step of performing library construction for the above-mentioned enriched DNA to obtain a sequencing library, different target regions can further be selected for sequencing, depending on detection purposes of different samples. During the actual operation of the present invention, in the above-mentioned detection device, the step of performing target region sequencing on the sequencing library to obtain a sequencing library containing the target regions is also included after obtaining the sequencing library and before performing the high-throughput sequencing. In a more preferred embodiment of the invention, the step of performing exon capture hybridization on the sequencing library is added, so that the subsequent high throughput sequencing is performed only for exons. Since introns are usually cleaved off during the transcription process of a gene, and exons are regions encoding a protein, only performing exon sequencing can increase an effective data volume and improve efficiency of sequencing. After obtaining a sequencing library for exons, it is also possible to detect mutations of known monogenic diseases within specific target regions depending on detection purposes and/or detection samples, or to detect all mutations in the sequencing data as entirety.

In the above-mentioned preferred embodiment of the invention, different methods or reagents may be selected for capture depending on the target regions to be captured. For example, a capture kit from Roche NimbleGen, US, or a self-made kit or a commercially available kit from other companies with a similar function can be used to perform target region sequencing.

Beneficial effects of the present invention will be further described below in conjunction with examples.

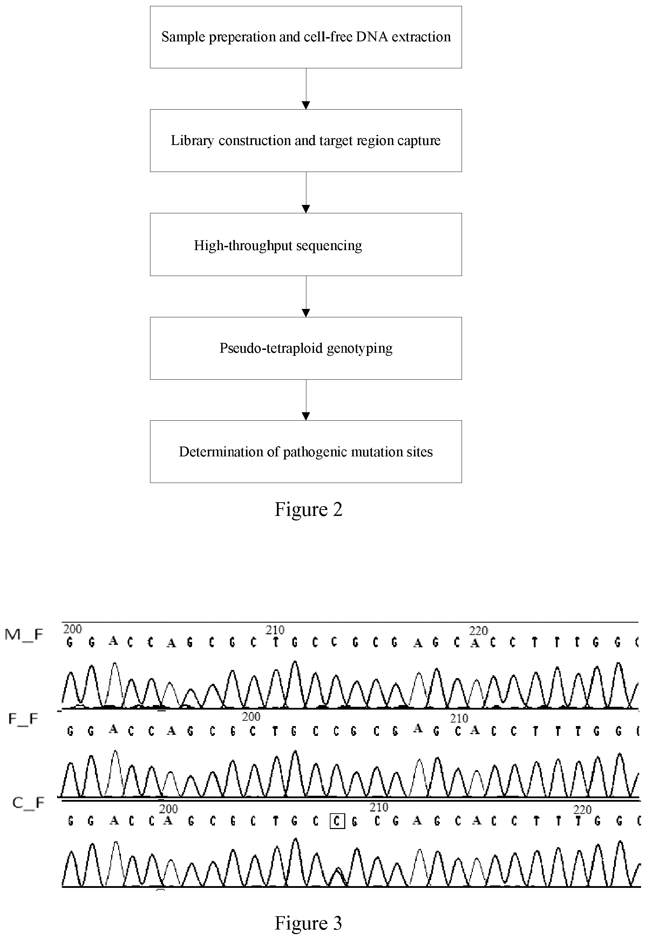

It should be noted that Example 1 is carried out in accordance with the flowchart shown in Figure 2. All reagents used in the following examples are from NEB unless otherwise specified; and the methods used can be carried out by conventional methods in the art unless otherwise specified.

## Example 1. A method for detecting gene mutations

Experiment 1: Sample preperation and cell-free DNA extraction

(1) Peripheral blood extacted from a pregnant woman was placed in a centrifuge for centrifugation at a speed of 1600 g for 10 min, and then the plasma was collected.

(2) After obtaining the peripheral blood plasma extacted from the pregnant woman, cell-free DNA in the plasma was extracted using the QIAamp DNA Blood Mini Kit (Qiagen, Germany, catlog # 51106) by following the method written in the user's manual.

Experiment 2: Capture enrichment and library construction

2.1 End-repair of cell-free DNA in plasma of the pregnant woman

Experimental objective: since the cell-free DNA extracted from the plasma of the pregnant woman are double-stranded DNA fragments which are either blunt-ended or contain 3' or 5' overhangs. In this step, the overhangs were phosphorylated to blunt ends by T4 DNA polymerase, a large fragment of *E. coli* DNA polymerase I (Klenow fragment) and polynucleotide kinase T4. The 3' to 5' exonuclease activity of the large fragment of DNA polymerase I removes the 3' overhangs and the T4 DNA polymerase activity fills the 5' overhangs. Eventually the cell-free DNAs have blunt ends.

Experimental materials, reagents and instruments: cell-free DNA of Experiment 1; a mixture of dNTPs (10 mM); T4 DNA polymerase (3 units/$\mu$L); Klenow fragment (5 units/$\mu$L); T4 PNK (T4 polynucleotide kinase, 10 units/$\mu$L) and PNK buffer; magnetic beads for DNA purification; and a PCR instrument.

Experimental procedure:

A. Formulating the following reaction system:

| | |
|---|---|
| Plasma DNA of a pregnant woman | 40 $\mu$L |
| A mixture of dNTPs (10 mM) | 2 $\mu$L |
| T4 DNA polymerase | 1 $\mu$L |
| Klenow fragment | 1 $\mu$L |
| T4 PNK (T4 polynucleotide kinase) | 1 $\mu$L |
| PNK buffer | 5 $\mu$L |
| Total volume | 160 $\mu$L |

B. Incubating in a PCR instrument at 20°C for 30 minutes;

C. Purifying the reaction product with magnetic beads, and eluting with 19.5 $\mu$L elution buffer (EB).

2.2 Adding base "A" at a 3' end of cell-free DNA fragments

Implementation objective: since subsequent adapter sequences contain a single "T" base at the 3' end needs to be ligated with the end-repaired cell-free DNA fragments, it is therefore necessary to first add a complementary "A" bases at the 3' end of the end-repaired fragments. This step is accomplished by using the Klenow fragment absent of 3' to 5' exonuclease activity.

Experimental materials, reagents and instruments: end-repaired cell-free DNA; Klenow buffer (10×); dATP (1 mM); Klenow fragment (absent of 3' to 5' exonuclease activity, 5 U/μL); and a PCR instrument.

Experimental procedure:

A. Formulating the following reaction system:

| End-repaired cfDNA | 19. 5 μL |
|---|---|
| Klenow buffer (10×) | 2. 5 μL |
| dATP (1 mM) | 2. 5 μL |
| Klenow fragment | 0.5 μL |
| Total volume | 25 μL |

B. Incubating in a PCR instrument at 37°C for 30 minutes.

2.3 Ligating adapters to both ends of the DNA fragments

Experimental objective: in order to enable the DNA fragment with added "A" to be specifically amplified in the subsequent PCR steps, it is necessary to use a DNA ligase to ligate specific adapters (i.e. an annealing product of each of adapter sequence 1 and adapter sequence 2) at both ends of the DNA.
Experimental materials, reagents and instruments: DNA with added "A" base; DNA ligase buffer (2×); DNA ligase (1 U/μL); adapter sequence 1 and adapter sequence 2; a PCR instrument; and magnetic beads for DNA purification.
The sequence of the adapter sequence 1 is
SEQ ID NO: 1: 5' P-GATCGGAAGAGCACACGTCT-3';
The sequence of the adaptor sequence 2 is SEQ ID NO: 2:
5' P-ACACTCTTTCCCTACACGACGCTCTTCCGATCT-3' (Illumina).

Experimental procedure:

A. Formulating the following reaction system:

| DNAs obtained in step 2.2 | 23 μL |
|---|---|
| DNA ligase buffer (2×) | 25 μL |
| DNA ligase (1 unit/μL) | 1 μL |
| Adapter (20 pmol/μL) | 1 μL |
| Total volume | 50 μL |

B. Incubating in a PCR instrument at 20°C for 15 minutes.
C. Purifying the reaction product with magnetic beads, and eluting with 38.2 μL elution buffer (EB).

2.4 PCR amplification of the DNA fragments with adapters ligated to both ends.

Experimental objective: PCR amplification was carried out on the DNA fragment modified by ligating adapters to both ends. On the one hand, the complementary sequence of sequencing primer attatched to both ends of the treated cell-free DNA fragments could be filled during the process of PCR, and on the other hand, sufficient amount of the DNA fragments could be abtained to continue the subsequent sequencing steps.
Experimental materials, reagents and instruments: DNA fragments with adapters at each of the two ends; 10×Pfx DNA polymerase amplification buffer; a mixture of dNTPs (10 mM); MgSO$_4$ (50 mM); PCR primer 1 (10 pmol/pL); PCR primer 2 (10 pmol/μL); and Pfx DNA polymerase (2.5 U/μL).
The sequence of PCR primer 1 is SEQ ID NO: 3:

5'-AATGATACGGCGACCACCGAGATCTACACTCTTTCCCTACACGACGCTCTTCCG
ATCT-3';

The sequence of PCR primer 2 is SEQ ID NO: 4:

5'-CAAGCAGAAGACGGCATACGAGATCGTGATGTGACTGGAGTTCAGACGTGTG
CTCTTCCGATCT-3'.

Experimental procedure:

A. Formulating the following reaction system:

| | |
|---|---|
| DNAs obtained in step 2.3 | 38.2 μL |
| 10×PFx DNA polymerase amplification buffer | 5 μL |
| A mixture of dNTPs (10 mM) | 2 μL |
| MgSO$_4$ (50 mM) | 2 μL |
| PCR primer 1 (10 pmol/μL) | 1 μL |
| PCR primer 2 (10 pmol/μL) | 1 μL |
| PFx DNA polymerase | 0.8 μL |
| Total volume | 50 μL |

B. Performing amplification according to the following PCR procedures: step one: incubating at 94°C for 2 minutes; step two: denaturation at 94°C for 15 seconds; annealing at 62°C for 30 seconds; extension at 72°C for 30 seconds, and repeat step two for 15 cycles; step three: incubating at 72°C for 10 minutes; and step four, finishing the reaction, and preserving at 4°C.
C. Purifying the reaction product with magnetic beads and eluting with ddH$_2$O.
D. Completing library preparation, and measuring the concentration by Agilent's DNA detector 2100 and the concentration was measured as 21.34 ng/μL.

Experiment 3: Target region capture

3.1 Library hybridization

After the library was quantified, exon capture hybridization was performed using the capture kit SeqCap EZ Human Exome+UTR Kit (Cat# 06740308001) from Roche NimbleGen, USA.

Experimental materials, reagents: DNA library; SeqCap.EZ Exome+UTR.Library; Cot DNA; SeqCap EZ Hyb and Wash Kit; HE oligo sequence and TS-INV-HE index oligo sequence;
wherein, the HE oligo sequence is SEQ ID NO: 5:

5'-AATGATACGGCGACCACCGAGATCTACACTCTTTCCCTACACGACGCTCTTCCG
ATCT-3'.

The TS-INV-HE index oligo sequence is SEQ ID NO: 6:

5'-CAAGCAGAAGACGGCATACGAGATCGTGATGTGACTGGAGTTCAGACGT
GTGCTCTTCCGATCT-3'.

Experimental procedure:

A. Formulating the following reaction system:

| Reagent | Amount |
|---|---|
| DNA library obtained in step 2.4 | 1 $\mu$g |
| Cot DNA | 5 $\mu$g |
| HE oligo | 1000 pmol (1 $\mu$L of 1000$\mu$M) |
| TS-INV-HE index oligo | 1000 pmol (1 $\mu$L of 1000$\mu$M) |

B. Drying at 56°C with a vacuum concentrator after finishing the above procedure.
After evaporating samples to dry, adding 7.5$\mu$L 2×Hybridization Buffer and 3$\mu$L Hybridization Component A, mixing uniformly, and performing denaturation at 95°C for 10 minutes.
C. After finishing the denaturation, transferring the above-mentioned mixture to 0.2 mL PCR tube with a convex cap, and adding 4.5 $\mu$L SeqCap EZ Exome+UTR Library. Vortexing for 3 seconds, mixing thoroughly, and centrifuging at the maximum speed for 10 seconds.
D. Placing a sample mixture to be hybridized at 47°C for heating for 64 to 72 hours for hybridization.

3.2 Elution and recovery of captured DNA

Experimental reagents: streptavidin Dynabeads; 1×Stringent Wash Buffer; 1×Wash Buffer I; 1×Wash Buffer II; 1×Wash Buffer III and ddH$_2$O.

Experimental procedure:

A. Transferring the sample library to a 0.2 mL PCR tube containing streptavidin Dynabeads, pipetting up and down 10 times and mixing well; then placing the 0.2 mL PCR tube in a heating module and incubate at 47°C for 45 minutes. During the incubation, vortexing the tube every 15 minutes to mix the teagents, so that the DNA fragments could bind to the magnetic beads.

B. After incubating for 45 min, adding 100 $\mu$L 1×Wash Buffer I at 47°C to a 15 $\mu$L captured DNA sample. Vortexing and mixing uniformly for 10 sec. Transferring all components in the 0.2 mL PCR tube to a 1.5 mL centrifuge tube. Placing the centrifuge tube on a magnetic stand to gather the magnetic beads, and discarding the supernatant.

Then removing the 1.5 mL centrifuge tube from the magnetic stand, and adding 200 $\mu$L 1×Stringent Wash Buffer preheated at 47°C. Pipetting up and down 10 times to mix uniformly. After mixing, placing the sample on a heating module at 47°C for 5 minutes, and washing twice with a 1×Stringent Wash Buffer at 47°C. Placing the 1.5 mL centrifuge tube on a magnetic stand again, and discarding a supernatant.
Adding 200 $\mu$L 1×Wash Buffer I at room temperature to the above-mentioned 1.5 mL centrifuge tube, and vortexing and mixing uniformly for 2 min. Placing the centrifuge tube on a magnetic stand and discarding the supernatant.
Adding 200 $\mu$L 1×Wash Buffer II at room temperature to the above-mentioned 1.5 mL centrifuge tube, and vortexing and mixing uniformly for 1 min. Placing the centrifuge tube on a magnetic stand and discarding a supernatant.
Adding 200 $\mu$L 1×Wash Buffer III at room temperature to the above-mentioned 1.5 mL centrifuge tube, and vortexing and mixing uniformly for 30 sec. Placing the centrifuge tube on a magnetic stand and discarding a supernatant.
Removing the 1.5 mL centrifuge tube from the magnetic stand, adding 50 $\mu$L ddH$_2$O to elute the magnetic bead-captured sample. Storing the magnetic bead-sample mixture at -20°C.

3.3 PCR amplification of captured DNA

Experimental objective: due to the very low concentration of DNA samples captured, PCR amplification is needed to meet the requirements of subsequent experiments.
Experimental materials and reagents: captured DNA by hybridization; 10×Pfx DNA polymerase amplification buffer; a dNTP mixture (10 mM); MgSO$_4$ (50 mM); PCR primer 3 (10 pmol/$\mu$L) (Invitrogen); PCR primer 4 (10 pmol/$\mu$L); and Pfx DNA polymerase (2.5 U/$\mu$L).

The sequence of the PCR primer 3 is SEQ ID NO: 7:

5'-AATGATACGGCGACCACCGAGATCTACACTCTTTCCCTACACGACGCTCTT CCGATCT-3';

The sequence of the PCR primer 4 is SEQ ID NO: 8:

5'-CAAGCAGAAGACGGCATACGAGATCGTGATGTGACTGGAGTTCAGACGT GTGCTCTTCCGATCT-3'.

Experimental procedure:

A. Formulating the following reaction system:

| | |
|---|---|
| Captured DNA | 38.2 $\mu$L |
| 10×PFX DNA polymerase amplification buffer | 5 $\mu$L |
| A mixture of dNTPs (10 mM) | 2 $\mu$L |
| MgSO$_4$ (50 mM) | 2 $\mu$L |
| PCR primer 1 (10 pmol/$\mu$L) | 1 $\mu$L |
| PCR primer 2 (10 pmol/$\mu$L) | 1 $\mu$L |
| PFX DNA polymerase | 0.8 $\mu$L |
| Total volume | 50 $\mu$L |

B. Performing amplification according to the following PCR procedures: step one: incubating at 94°C for 2 minutes; step two: denaturation at 94°C for 15 seconds; annealing at 62°C for 30 seconds; extension at 72°C for 30 seconds, repeat step two for 13 cycles; step three: incubating at 72°C for 10 minutes; and step four, finishing the reaction, and preserving at 4°C.
C. Purifying the reaction product with magnetic beads and eluting with 30$\mu$L elution buffer ddH$_2$O.
D. After preparing the library, and measuring the concentration using Agilent's DNA detector 2100 and the concentration was measured as 13.60 ng/$\mu$L.

Experiment 4: Loading and sequencing

The DNA molecules in the sequencing library were made into DNA clusters using cBot instrument from Illumina, and the resulting DNA clusters were subjected to 100 cycles of double-end sequencing on an Illumina Hiseq 2000 (or Illumina HiSeq 2500) sequencer.
The raw image data files obtained by high-throughput sequencing (Illumina HiSeqTM2000) were converted by the CASAVA Base Calling raw sequenced sequence (Sequenced Reads) which were also known as Raw Data or Raw Reads. The results were saved as a FASTQ (abbreviated as fq) file format containing the sequence information of the sequenced sequences (reads) and the corresponding sequencing quality information.

Experiment 5: Processing of raw sequencing data

5.1 Filtering out unqualified reads

Raw reads obtained by sequencing contain reads with adapters and reads low sequencing quality (over 50% nucleotide bases have a sequencing quality score of Q ≤ 5 in a read). In order to ensure the quality of the analysis result, the raw reads should be filtered to obtain reads with qualified sequencing quality and with adapters removed (also known as clean reads), and subsequent analysis is based on the filtered reads. The following sequences are filtered out: (1) reads containing N at a ratio of greater than 5%; (2) low-quality reads (nucleotide bases with a quality value of Q ≤ 5 accounts for 50% or more of the entire read length); and (3) reads contaminated with adapters.

The raw data statistics for the samples are shown in Table 2. The modified Q30 bases rate (%) indicates the proportion of bases with a quality value greater than 30 (an error rate of less than 0.1%) in the total sequence after filtration. The larger the value, the better the sequencing quality. Generally, if the index is greater than 85%, the sequencing quality is considered qualified. If it is less than 85%, then re-sequencing is required.

Table 2:

| Raw reads | Clean reads | Effect rate (%) | Q30 (%) |
|---|---|---|---|
| 140,070,440 | 136,958,780 | 97. 78 | 86. 76 |

Effect rate (%): The percentage of reads obtained by dividing the clean reads to the raw reads. The clean reads were obtained by removing the following ones in the raw reads: 1. Low-quality reads, which nucleotide bases with a quality value of Q $\leq$5 accounts for 50% or more of the entire read length; 2. reads containing N at a ratio of greater than 5%; and 3. reads with adapter contamination.

5.2 Mapping quality control

By mapping filtered clean reads to a reference genome (HG19, NCBI built 37) using the mapping software BWA (bwa-0.7.5a), the mapping results are subjected to mapping quality control to obtain a mapping file.

The quality control points comprise the data mapping rate, capture specificity, target region sequencing depth, target region sequencing depth distribution, PCR duplication rate and the like. The results of the mapping quality control are shown in Table 3.

Table 3:

| Duplication rate (%) | Mapping rate (%) | Capture specificity | Target region sequencing depth (X) (Target Average depth) |
|---|---|---|---|
| 5. 54 | 97. 44 | 72. 38 | 139. 78 |

In the above-mentioned Table 3, the capture specificity means that reads are completely mapped to a target region, and reads are partially in the target region and are partially outside of the region; the Target Average depth (X) refers to the depth of a target region; the duplication rate (%) involves reads that are duplicated due to PCR amplification; the mapping rate (%) refers to a ratio of the reads mapped to hg19 reference genome in the raw data using BWA, and generally 90% or more can be considered as normal results.

Then, based on the above-mentioned mapping file, useless data (such as duplication reads, etc.) are removed, and a set of sites with the nucleotide sequence different from the reference genome are obtained;

Finally, statistic analysis of the sequencing results of the above-mentioned differential base sites are performed, for example, 40 As and 60 Ts being detected at a site, and some other information such as the location of a site.

**Experiment 6: Mixed genotyping of pseudo-tetraploid**

Target areas of the pregnant woman and fetus is directly genotyped only by genetic information derived from peripheral blood of the pregnant woman as mentioned above. According to the pseudo-tetraploid genotyping model of the present invention, a mixed genotype of a pseudo-tetraploid composed of a genotype of the pregnant woman and a genotype of the fetus is deduced to obtain genotypes of the pregnant woman and the fetus at each corresponding site. The following content takes the specific situation of a site as an example to illustrate the process of deducing the genotypes of the pregnant woman and the fetus.

If at the site, the reference allele is detected 50 times, the mutant allele is detected 8 times, the fetal concentration is 8%, the population mutation frequency is 0.03, and $G_{j*}$ in the formula (4) is $G_4$, then the formula (4') is obtained

$$\varphi_j = \frac{P(S_i \mid G_{ij})P(G_{ij})}{P(S_i \mid G_{i4})P(G_{i4})}$$

$$(4')$$

In combination with the formula (4') to the formula (7) and Table 1, a probability ratio $\varphi$ of the 7 mixed genotypes and the fourth mixed genotype is calculated, thereby obtaining a corresponding mixed genotype with the highest probability ratio $\varphi$. The specific calculation process is as follows:
when the mixed genotype is AAAA,

$$\varphi_1 = \frac{P(S_i \mid G_{i1})P(G_{i1})}{P(S_i \mid G_{i4})P(G_{i4})} = \frac{\binom{50+8-1}{8-1} 0^8 (1-0)^{50} \times (1-0.03)^2 (1-0.03)^2}{\binom{50+8-1}{8-1} 0.5^8 (1-0.5)^{50} \times (2 \times 0.03 \times (1-0.03))^2} ;$$

when the mixed genotype is AAAB,

$$\varphi_2 = \frac{P(S_i \mid G_{i2})P(G_{i2})}{P(S_i \mid G_{i4})P(G_{i4})} = \frac{0.04^8 (1-0.04)^{50} \times (1-0.03)^2 \times 2 \times 0.03 \times (1-0.03)}{0.5^8 (1-0.5)^{50} \times (2 \times 0.03 \times (1-0.03))^2} ;$$

when the mixed genotype is ABAA,

$$\varphi_3 = \frac{P(S_i \mid G_{i3})P(G_{i3})}{P(S_i \mid G_{i4})P(G_{i4})} = \frac{0.46^8 (1-0.46)^{50} \times (1-0.03)^2 \times 2 \times 0.03 \times (1-0.03)}{0.5^8 (1-0.5)^{50} \times (2 \times 0.03 \times (1-0.03))^2} ;$$

when the mixed genotype is ABAB,

$$\varphi_4 = \frac{P(S_i \mid G_{i4})P(G_{i4})}{P(S_i \mid G_{i4})P(G_{i4})} = 1 ;$$

when the mixed genotype is ABBB,

$$\varphi_5 = \frac{P(S_i \mid G_{i5})P(G_{i5})}{P(S_i \mid G_{i4})P(G_{i4})} = \frac{0.54^8 (1-0.54)^{50} \times (0.03)^2 \times 2 \times 0.03 \times (1-0.03)}{0.5^8 (1-0.5)^{50} \times (2 \times 0.03 \times (1-0.03))^2} ;$$

when the mixed genotype is BBAB,

$$\varphi_6 = \frac{P(S_i \mid G_{i6})P(G_{i6})}{P(S_i \mid G_{i4})P(G_{i4})} = \frac{0.96^8 (1-0.96)^{50} \times (0.03)^2 \times 2 \times 0.03 \times (1-0.03)}{0.5^8 (1-0.5)^{50} \times (2 \times 0.03 \times (1-0.03))^2} ;$$

and when the mixed genotype is BBBB,

$$\varphi_7 = \frac{P(S_i \mid G_{i7})P(G_{i7})}{P(S_i \mid G_{i4})P(G_{i4})} = \frac{1^8 (1-1)^{50} \times (0.03)^2 (0.03)^2}{0.5^8 (1-0.5)^{50} \times (2 \times 0.03 \times (1-0.03))^2} ;$$

All of the $\varphi$ values are compared, obtaining $\varphi_2 > \varphi_3 > \varphi_4 > \varphi_5 > \varphi_6 > \varphi_7 = \varphi_1$, and therefore the most likely mixed genotype of this site is type AAAB. Further, it is deduced that the genotype of the pregnant woman is AA, and the genotype of the fetus is AB. According to the above-mentioned principle, a mixed genotype of each of all the variant sites (SNP sites) in the mapping result file is obtained, thereby the genotype of the fetus is obtained.

Experiment 7: Determination of pathogenic mutation sites

7.1 Filtering out non-pathogenic mutation sites

A fetal genotype at each variant site obtained from the results of pseudo-tetraploid typing is compared with the following databases, respectively, and the variant sites fullfiling the following criteria in the databases are filtered out: (1) high-frequency mutations in the dbSNP135 public database; and (2) polymorphic sites in the Freq 1000g2012feb (thousand human genome) database. (3) The mutation sites of synonymous mutations, nonsense mutations and non-conserved regions are filtered out according to the mutation prediction software. The sites that appeared in all of the above-mentioned three screening conditions are excluded to obtain fetal specific variant sites, as shown in Table 4.

Table 4:

| Total number of SNPs | Non-synonymo us mutation | ASN_freq<0. 05 site | dbSNP | Differential base site |
|---|---|---|---|---|
| 111407 | 100046 | 8622 | 100501 | 1049 |

7.2 Based on the published literatures and clinical data, pathogenic mutations are determined from the above-mentioned filtered mutation sites.

**Example 2. An example of non-invasive single-gene defect diagnosis of an osteogenesis imperfecta for a fetus**

Sample information: a pregnant woman, 28 years old, gravida 3 para 0, with regular menstrual of 5-6 days, and a menstrual cycle of 29 days. The last menstrual period was March 27, 2012, and the expected date of birth was January 4, 2013. She conceived naturally, had no history of fever, rash or the like during early pregnancy, and had no history of exposure to radiation or poisons. At gestational week 13, *Toxoplasma gondii*, rubella, cytomegalovirus, and herpes simplex virus were all test negative; at gestational week 14+, width of nuchal translucency (NT) of the fetus detected by B-mode ultrasound is 0.14 cm; and at gestational week 17+, 21-trisomy risk probability was < 1:50000 and 18-trisomy risk probability was < 1:50000 idicated by the serological screening. At gestational week 26+, B-mode ultrasound in a healthcare hospital suggested dysplasia of fetal femurs and tibias; and at gestational week 26+, reexamination through B-mode ultrasound suggested that fetal skull was thin with reduced echo, the length of bilateral femurs was 3.3 cm and bent into an angle, and the tibias and fibulas were also bent into an angle. It was considered that the fetal femurs, and the tibias and fibulas formed angles. At gestational week 30+, the pregnant woman carried out genetic mutation analysis using the method of the present invention, and the fetus wad diagnosed to have osteogenesis imperfecta and the pregnant women was recommended to terminate the pregnancy.

10 ml of peripheral blood was drawn from the pregnant woman, and according to the method of Example 1, cell-free DNA was extracted, captured and enriched, and the enriched DNA was subjected to high-depth sequencing through the HiSeq platform.

Pseudo-tetraploid typing: the sequencing result was subjected to quality control, low-quality data were filtered out, the remaining data were mapped to the genome, and according to the mapping result, the fetal genotyping information was deduced through the pseudo-tetraploid typing model of the present invention, and screened for whether it is related to osteogenesis.

Mutation site screening: 111407 raw mutations were filtered according to the steps in Example 1 for mutation sites, and finally 7 mutations were obtained. Literature review and clinical data review were performed on the screened 7 mutations, and one mutation was finally determined (COL1A1:NM_000088:c.G2596A:p.G866S) as a pathogenic mutation leading to osteogenesis imperfecta.

Verification of results: the fetal umbilical cord blood sample and peripheral blood samples of the pregnant woman and the fetal father were used to verify the pathogenic mutation obtained for the sample of the present example, and the results are shown in Figure 3 (in Figure 3, MF, FF or C-F represents the sense strand of gene of the mother, the father, and the fetus respectively). As shown in Figure 2, the fetus truly contains the pathogenic mutation at this site.

**Example 3. Verification of the pseudo-tetraploid typing model of the present invention.**

The above-mentioned peripherals blood sample of the pregnant woman is detected and analyzed by the pseudo-tetraploid typing model of the present invention, meanwhile, the somatic cell detection method of "a fetal cord blood sample + a peripheral blood sample of the pregnant woman" is used to verify and evaluate the validity of the pseudo-tetraploid typing model of the present invention.

In combination with the somatic cell sequencing result of the pregnant woman and the somatic cell sequencing result of the cord blood sample, comparing with the result of pseudo-tetraploid genotyping was compared and the accuracy of the method of the present invention are shown in Table 5 below; and the detection rate obtained by comparing with the somatic cell sequencing result of a pregnant woman and the somatic cell sequencing result of a cord blood sample in the prior art is shown in Table 6.

Table 5: Mixed genotyping of pseudo-tetraploid and detection rates

| Mixed genotype | Number of matched sites | Number of unmatched sites [A] | Total number of sites [A] | Accuracy /% |
|---|---|---|---|---|
| AAAA | 89078219 | 2056 | 89080275 | 99. 99 |
| AAAB | 10157 | 5654 | 15811 | 64. 24 |
| ABAA | 9968 | 6051 | 16019 | 62. 23 |
| ABAB | 16203 | 11711 | 27914 | 58. 05 |
| ABBB | 4936 | 4077 | 9013 | 54. 77 |
| BBAB | 7274 | 1646 | 8920 | 81. 54 |
| BBBB | 28284 | 821 | 29105 | 97. 18 |
| Total (without AAAA) | 76822 | 29960 | 106782 | 71. 94 |

Notes: since AAAA does not contain mutant allele B, the number of sites for this genotype is not counted in the total number of sites. The total number of sites in the above-mentioned table is the sum of the number of mapped base sites in the mother + the number of mapped base sites in the fetus by somatic cell sequencing for the mother and cord blood respectively (each of the repeatitive sites in mother and her fetus was only counted once for calculation).

The total number of sites [A] represents the total number of positive sites detected using the pseudo-tetraploid genotyping model of the present invention.

Number of matched sites is the number of true positive sites, representing the number of sites in the above-mentioned total number of sites [A] which are determined by the pseudo-tetraploid typing model of the present invention with true mutations. (The somatic cell sequencing performed using mother's blood and cord blood was consider as the gold standard for maternal and fetal genotype detection, and the method of determining maternal and fetal genotypes by the pseudo-tetraploid model is a subject method, and by comparing the subject method of the present invention with the gold standard, the site with consistent result is counted as a true negative or true positive site, and the inconsistent site is recorded as a false positive or false negative site.)

Number of unmatched sites is the number of false negative sites, representing the number of sites that are not determined by the pseudo-tetraploid typing of the present invention in the total number of sites [A].

Positive detection accuracy is measured as an index for evaluating the subject detection method, pseudo-tetraploid in this case. It is cauculated as true positive/(true positive + false negative) $\times$ 100%, i.e. a ratio of the number of true positive sites detected by pseudo-tetraploid method to the total number of sites [A].

Table 6: Somatic cell detection results and the detection rate

| Mixed genotype | Number of matched sites | Number of unmatched sites [B] | Total number of sites [B] | Detection rate/% |
|---|---|---|---|---|
| AAAA | 89078219 | 10897 | 89089116 | 99. 99 |
| AAAB | 10157 | 1808 | 11965 | 84. 89 |
| ABAA | 9968 | 5820 | 15788 | 63. 14 |
| ABAB | 16203 | 8872 | 25075 | 64. 62 |
| ABBB | 4936 | 2897 | 7833 | 63. 02 |
| BBAB | 7274 | 575 | 7849 | 92. 67 |
| BBBB | 28284 | 1384 | 29668 | 95. 34 |
| Total (without AAAA) | 76822 | 21356 | 98178 | 78. 25 |

The detection accuracy is the ratio of the number of positive sites detected by the subject method, pseudo-tetraploid in this case, to the number of true positive sites detected by the gold standard. In Table 6 above, the total number of sites [B] is the number of sites that have true positive mutations as determined by a somatic cell sequencing result of a pregnant woman and a somatic cell sequencing result of a cord blood sample. Number of matched sites is the number of true positive sites detected using the pseudo-tetraploid typing model. Number of unmatched sites is the number of true positive sites (i.e. the number of false negative sites) that were undetected by the pseudo-tetraploid typing model. Thus, the true positive/(false negative + true positive) $\times$ 100% in Table 6 represents the detection rate.

**Example 4**

A device for detecting gene mutations as defined in the claims comprises:
a detection module for performing high-throughput sequencing of cell-free DNAs in peripheral blood of a pregnant woman to obtain sequencing data. It comprises instruments for sequencing the cell-free DNAs in the peripheral blood of the pregnant woman which include cBot instrument from Illumina and Genome AnalXzer from Illumina, HiSeq2000 sequencer or HiSeq2500 sequencer or SOLiD series of sequencers from ABI.

Preferably, the detection module further comprising a region capture sub-module for performing target region capture on the sequencing library constructed from the enriched cell-free DNAs to obtain a sequencing library for high-throughput sequencing.

An alignment module for aligning the sequencing data with a reference genomic sequence to obtain SNP sites; a target mixed genotype determination module for performing mixed genotyping for each of the SNP sites using a Bayesian model and an initial fetal concentration f to obtain a mixed genotype with the maximum probability among seven mixed genotypes for each of the SNP sites, and taking the mixed genotype with the maximum probability as a target mixed genotype of each of the SNP sites; wherein the mixed genotype refers to pseudo-tetraploid genotypes formed by genotypes of the pregnant woman and the fetus, the mixed genotype is any one of seven types consisting of AAAA, AAAB, ABAA, ABAB, ABBB, BBAB, and BBBB, and are sequentially numbered as type 1, type 2, type 3, type 4, type 5, type 6 and type 7, where A represents a reference allele of each of the SNP sites, and B represents a mutant allele of each of the SNP sites; a target mixed genotype of fetus at each of the SNP sites is deduced by performing mixed genotyping according to conditional probability and the Bayesian model.

A mutation site screening module is used to screen a mutation site from various SNP sites according to the genotype of each of the SNP sites of the fetus.

Preferably, the mutation site screening module comprises: a high-frequence polymorphic site filtration sub-module for filtering out polymorphic sites with a high occurance frequency in the human population in each of the SNP sites of a fetal genotype to obtain preliminary candidate mutation sites; for example, the high-frequence polymorphic sites in the human population are removed using the dnSNP135 public database and the Freq_1000g2012feb (thousand human genome) database which have been currently collated by the medical community, and the specific SNP sites of the fetus are obtained, and then the sites which could actually cause gene mutations are screened by the gene mutation site screening sub-module.

A gene mutation site screening sub-module is used for filtering out SNP sites, which result in synonymous mutations and nonsense mutations and occur in a non-conserved region, from the preliminary candidate mutation sites to obtain candidate mutation sites. The module can use a mutation prediction module commonly used in the art for performing harmful mutation screening. For example, ANNOVAR module can screen whether the mutation causes an amino acid change, that is, whether the mutation is nonsynonymous, and can also screen whether the mutation occurs in a conserved sequence region.

A Literature and clinical data review sub-module is used for performing screening on the candidate mutation sites to obtain the pathogenic mutation site that has been recorded in the literature and clinical data. SNP sites which have been screened by the mutation prediction module and pathogenic sites which are retrieved from existing databases and literatures are aligned, so as to find the site information associated with a monogenic disease and perform corresponding interpretation. The presence or absence of a hot spot mutation site leading to a known monogenic disease can be detected, and non-hot spot mutation sites of a known monogenic disease and an unreported potential pathogenic gene and its mutation sites can also be detected.

From the above description, it can be seen that the method, and device for the non-invasive prenatal gene mutation diagnosis of the present invention can infer the fetal genotype and determine whether the genotype would cause a corresponding disease, only by the genetic information of cell-free DNA in the peripheral blood of the pregnant woman. The genetic information of the fetus's father or mother is not required. It simplifies the process of non-invasive single-gene defect detection and reduces the cost of the test. In addition, the present invention can not only detect a specific single-gene defect, but also can detect multiple single-gene defects simultaneously.

It will be apparent to those skilled in the art that some of the modules, elements or steps of the present application described above may be implemented by a general-purpose computing device, and they may be integrated on a single computing device or distributed across a net composed of multiple computing devices. Alternatively, they may be implemented by program codes executable by the computing device, and accordingly they may be stored in a storage device for execution by the computing device; or they may be separately fabricated into individual integrated circuit modules, or multiple modules or steps may be implemented by fabricating them as a single integrated circuit module.

As such, the present application is not limited to a combination of any particular hardware and software.

**Claims**

1. A computer-implemented method for detecting mutations in a fetal genome, wherein the method comprises the steps of:

   step A, obtaining sequencing data from high-throughput sequencing of cell-free DNAs in maternal peripheral blood;
   step B, aligning the sequencing data with those of a reference genome to obtain SNP sites;
   step C, performing mixed genotyping for each of the SNP sites using a Bayesian model and an fetal concentration $f$ to obtain a mixed genotype with the maximum probability among seven mixed genotypes for each of the SNP sites, and taking the mixed genotype with the maximum probability as a target mixed genotype of each of the SNP sites, wherein performing mixed genotyping comprises the steps of:
   calculating

$$\varphi_j = \frac{P(G_{ij} \mid S_i)}{P(G_{ij*} \mid S_i)} = \frac{P(S_i \mid G_{ij})P(G_{ij})}{P(S_i \mid G_{ij*})P(G_{ij*})} \tag{4}$$

   wherein, $\varphi_j$ represents the ratio of the probability of the mixed genotype $G_j$ at the i-th SNP site to a probability of the mixed genotype $G_{j*}$ at the i-th SNP site under the $S_i$ condition,
   wherein P($S_i$ | Gij) is calculated as follows:

$$P(S_i \mid G_{ij}) = \binom{k+r-1}{r-1} \times f(b)^r \times (1-f(b))^k$$

(7),

wherein r represents the number occurrence of a mutant allele at the i-th SNP site, k represents the number occurrence of a reference allele at the i-th SNP site,

wherein f(b) refers to the probability of the mutant allele in the mixed genotype, when the mixed genotype $G_{ij}$ is $G_{i1}$, the value of the $f(b)$ is 0; when the mixed genotype $G_{ij}$ is $G_{i2}$, the value of the $f(b)$ is $f/2$; when the mixed genotype $G_{ij}$ is $G_{i3}$, the value of the $f(b)$ is 0.5-$f/2$; when the mixed genotype $G_{ij}$ is $G_{i4}$, the value of the $f(b)$ is 0.5; when the mixed genotype $G_{ij}$ is $G_{i5}$, the value of the $f(b)$ is 0.5+$f/2$; when the mixed genotype $G_{ij}$ is $G_{i6}$, the value of the $f(b)$ is 1-$f/2$; and when the mixed genotype $G_{ij}$ is $G_{i7}$, the value of the $f(b)$ is 1; wherein the $f$ represents the fetal concentration,

wherein P(Gij) is calculated as follows:

$$\begin{cases} P(G' = AA) = (1-\theta)^2 \\ P(G' = AB) = 2\theta(1-\theta) \\ P(G' = BB) = \theta^2 \end{cases}$$

(6)

wherein $\theta$ is the population mutation frequency of the i-th SNP site, and

finding the mixed genotype with maximum occurrence probability among the seven mixed genotypes by using the formula

$$G = \arg\max(\varphi_j)$$

(5),

and

recording the mixed genotype with maximum occurrence probability as the mixed genotype with maximum probability at the i-th SNP site; and

step D, identifying from the fetal genotype in the mixed genotype pathogenic mutations;

wherein the mixed genotype refers to the pseudo-tetraploid genotype, which is composed of genotypes of the pregnant woman and the fetus, the mixed genotype is any one of seven types, AAAA, AAAB, ABAA, ABAB, ABBB, BBAB, and BBBB, , where A represents the reference allele of each SNP sites, and B represents the mutant allele of each SNP sites, wherein the seven types are sequentially numbered as type 1, type 2, type 3, type 4, type 5, type 6 and type 7, and

wherein the method does not include separate sequencing of paternal and/or maternal samples.

2. The method according to claim 1, wherein the fetal concentration is calculated by iteration as follows:

step 0: pre-estimating that fetal concentration $f$ is a defined value which is chosen from 5 to 20%; iteration:

step 1: inferring the fetal genotype according to the mixed genotyping as defined in claim 1, and calculating the fetal concentration $f'$ according to the $f(b)$ in the genotype;

step 2: calculating Δ$f$, wherein Δ$f$ = D($f$-$f'$);

step 3: if $\Delta f < \varepsilon$, the iteration ends, wherein $\varepsilon$ is less than or equal to 0.001; and

step 4: obtaining the fetal concentration $f$ = $f$(b);

wherein the function D( ) represents a distance function, which measures a difference between two variables; and wherein f(b) refers to the probability of the mutant allele in the mixed genotype, when the mixed genotype $G_{ij}$ is $G_{i7}$, the value of the $f(b)$ is 0; when the mixed genotype $G_{ij}$ is $G_{i2}$, the value of the $f(b)$ is $f/2$; when the mixed genotype $G_{ij}$ is $G_{i3}$, the value of the $f(b)$ is 0.5-$f/2$; when the mixed genotype $G_{ij}$ is $G_{i4}$, the value of the $f(b)$ is 0.5; when the mixed genotype $G_{ij}$ is $G_{i5}$, the value of the $f(b)$ is 0.5+$f/2$; when the mixed genotype $G_{ij}$ is

$G_{i6}$, the value of *the f(b)* is 1-*f*/2; and when the mixed genotype $G_{ij}$ is $G_{i7}$, the value of *the f(b)* is 1.

3. The method according to claim 1, wherein the steps of identifying from the fetal genotype in the mixed genotype pathogenic mutations comprises:

   filtering the polymorphic sites with a high incidence in the human population in a fetal genotype in the target mixed genotype of each of the SNP sites to obtain preliminary candidate mutation sites;
   filtering SNP sites of synonymous mutations and nonsense mutations and mutations occuring in non-conserved regions, from the preliminary candidate mutation sites to obtain candidate mutation sites; and
   performing a literature and database search for information associated with a monogenic disease on the candidate mutation sites to obtain the pathogenic mutations.

4. A device for detecting gene mutations, wherein the device comprises:

   a detection module for performing high-throughput sequencing of cell-free DNA in maternal peripheral blood to obtain sequencing data;
   an alignment module for aligning the sequencing data with a reference genomic sequence to obtain SNP sites;
   a target mixed genotype determination module for performing mixed genotyping at each SNP site using a Bayesian model and an initial fetal concentration *f* to obtain a mixed genotype with the maximum probability among seven mixed genotypes of each SNP site according to claim 1, and taking the mixed genotype with the maximum probability as the target mixed genotype of each of the SNP sites; and
   a mutation site screening module for identifying from the fetal genotype in the mixed genotype pathogenic mutations in the target mixed genotype of each of the SNP sites;
   wherein the mixed genotype refers to the pseudo-tetraploid genotypes, which is composed of genotypes of the pregnant woman and the fetus, the mixed genotype is any one of seven types, AAAA, AAAB, ABAA, ABAB, ABBB, BBAB, and BBBB, , where A represents a reference allele of each of the SNP sites, and B represents a mutant allele of each of the SNP sites, wherein the seven types are sequentially numbered as type 1, type 2, type 3, type 4, type 5, type 6 and type 7.

5. The device according to claim 4, wherein the mutation site screening module comprises:

   a high-incidence polymorphic site filtration sub-module for filtering out polymorphic sites with high incidence in the human population in a fetal genotype in the target mixed genotype of each of the SNP sites to obtain preliminary candidate mutation sites;
   a gene mutation site screening sub-module for filtering SNP sites of synonymous mutations, nonsense mutations and mutations occuring in non-conserved regions, from the preliminary candidate mutation sites to obtain candidate mutation sites; and
   a literature and clinical data review sub-module for performing a literature and database search for information associated with a monogenic disease on the candidate mutation sites to obtain the pathognic mutations.

## Patentansprüche

1. Computer-implementiertes Verfahren zum Nachweis von Mutationen in einem fötalen Genom, wobei das Verfahren die Schritte umfasst:

   Schritt A, Erhalten von Sequenzdaten aus Hochdurchsatz-Sequenzierung von zellfreier DNA in mütterlichem peripheren Blut;
   Schritt B, Vergleichen (aligning) der Sequenzierungsdaten mit jenen eines Referenzgenoms unter Erhalt von SNP Stellen;
   Schritt C, Durchführen gemischter Genotypisierung für jede SNP Stelle mit einem Bayes Modell und einer fötalen Konzentration *f* unter Erhalt eines gemischten Genotyps mit der maximalen Wahrscheinlichkeit von sieben gemischten Genotypen für jede der SNP Stellen, und Auswählen des gemischten Genotyps mit der maximalen Wahrscheinlichkeit als Ziel-gemischter Genotyp für jede der SNP Stellen, wobei das Durchführen der gemischten Genotypisierung die Schritte umfasst:
   Berechnen

$$\varphi_j = \frac{P(G_{ij} \mid S_i)}{P(G_{ij*} \mid S_i)} = \frac{P(S_i \mid G_{ij})P(G_{ij})}{P(S_i \mid G_{ij*})P(G_{ij*})}$$

(4)

wobei, $\varphi_j$ das Verhältnis der Wahrscheinlichkeit des gemischten Genotyps $G_j$ an der i-ten SNP Stelle zur Wahrscheinlichkeit des gemischten Genotyps $G_{j*}$ at the i-ten SNP Stelle unter der $S_i$ Bedingung bedeutet,
wobei P($S_i$ | Gij) wie nachstehend berechnet wird:

$$P(S_i \mid G_{ij}) = \binom{k+r-1}{r-1} \times f(b)^r \times (1 - f(b))^k$$

(7),

wobei r die Anzahl des Auftretens eines mutierten Allels an der i-ten SNP Stelle bedeutet, k die Anzahl des Auftretens eines Referenzallels an der i-ten SNP Stelle bedeutet,
wobei f(b) die Wahrscheinlichkeit des mutierten Allels in dem gemischten Genotyp bedeutet, wenn der gemischte Genotyp $G_{ij}$ der Genotyp $G_{i1}$ ist, ist der Wert von f(b) 0; wenn der gemischte Genotyp $G_{ij}$ der Genotyp $G_{i2}$ ist, ist der Wert von f(b) f/2; wenn der gemischte Genotyp $G_{ij}$ der Genotyp $G_{i3}$ ist, ist der Wert von f(b) 0.5-f/2; wenn der gemischte Genotyp $G_{ij}$ der Genotyp $G_{i4}$ ist, ist der Wert von f(b) 0.5; wenn der gemischte Genotyp $G_{ij}$ der Genotyp $G_{i5}$ ist, ist der Wert von f(b) 0.5+f/2; wenn der gemischte Genotyp $G_{ij}$ der Genotyp $G_{i6}$ ist, ist der Wert von f(b) 1-f/2; und wenn der gemischte Genotyp $G_{ij}$ der Genotyp $G_{i7}$ ist, ist der Wert von f(b) 1; wobei f die fötale Konzentration bedeutet,
wobei P(Gij) wie nachstehend berechnet wird:

$$\begin{cases} P(G' = AA) = (1-\theta)^2 \\ P(G' = AB) = 2\theta(1-\theta) \\ P(G' = BB) = \theta^2 \end{cases}$$

(6)

wobei $\theta$ die Populationsmutationsfrequenz der i-ten SNP Stelle bedeutet, und
Auffinden des gemischten Genotyps mit der maximalen Wahrscheinlichkeit des Auftretens unter den sieben gemischten Genotypen unter Verwendung der Formel

$$G = \arg\max(\varphi_j)$$

(5),

und
Festlegen des gemischten Genotyps mit maximaler Wahrscheinlichkeit des Auftretens als der gemischte Genotype mit maximaler Wahrscheinlichkeit an der i-ten SNP Stelle; und
Schritt D, Identifizieren von pathogenenen Muationen aus dem fötalen Genotyp in dem gemischten Genotyp;
wobei der gemischte Genotyp den Pseudo-Tetraploid Genotyp bedeutet, der zusammengesetzt ist aus den Genotypen der schwangeren Frau und des Fötus, wobei der gemischte Genotyp ein Typ ausgewählt aus den Typen AAAA, AAAB, ABAA, ABAB, ABBB, BBAB und BBBB ist, wobei A das Referenzallel an jeder der SNP Stellen darstellt, und B das mutierte Allel an jeder der SNP Stellen darstellt, wobei die sieben Typen fortlaufend als Typ 1, Typ 2, Typ 3, Typ 4, Typ 5, Typ 6 und Typ 7 nummeriert werden, und
wobei das Verfahren nicht unabhängig davon das Sequenzieren von väterlicher und/oder mütterlichen Proben umfasst.

2. Verfahren gemäß Anspruch 1, wobei die fötale Konzentration durch Iteration wie nachstehend berechnet wird:

Schritt 0: Abschätzen, dass die fötale Konzentration fein definierter Wert ausgewählt aus 5 bis 20 % ist;
Iteration:

Schritt 1: Ableiten des fötalen Genotyps gemäß dem in Anspruch 1 definierten gemischten Genotypisieren, und Berechnen der fötalen Konzentration $f'$ gemäß $f(b)$ in dem Genotyp;

Schritt 2: Berechnen von $\Delta f$, wobei $\Delta f = D(\,f - f')$;

Schritt 3: wenn $\Delta f < \varepsilon$, endet die Iteration, wobei $\varepsilon$ kleiner oder gleich 0.001 ist; und

Schritt 4: Erhalten der fötalen Konzentration $f = f\,(b)$;

wobei die Funktion D() eine Distanzfunktion bedeutet, die eine Differenz zwischen zwei Variablen bestimmt; und wobei f(b) die Wahrscheinlichkeit des mutierten Allels in dem gemischten Genotyp bedeutet, wenn der gemischte Genotyp $G_{ij}$ der Genotyp $G_{i1}$ ist, ist der Wert von $f(b)$ 0; wenn der gemischte Genotyp $G_{ij}$ der Genotyp $G_{i2}$ ist, ist der Wert von $f(b)$ $f/2$; wenn der gemischte Genotyp $G_{ij}$ der Genotyp $G_{i3}$ ist, ist der Wert von $f(b)$ 0.5-$f/2$; wenn der gemischte Genotyp $G_{ij}$ der Genotyp $G_{i4}$ ist, ist der Wert von $f(b)$ 0.5; wenn der gemischte Genotyp der Genotyp $G_{i5}$ ist, ist der Wert von $f(b)$ 0.5+$f/2$; wenn der gemischte Genotyp $G_{ij}$ der Genotyp $G_{i6}$ ist, ist der Wert von $f(b)$ 1-$f/2$; und wenn der gemischte Genotyp $G_{ij}$ der Genotyp $G_{i7}$ ist, ist der Wert von $f(b)$ 1.

3. Verfahren gemäß Anspruch 1, wobei der Schritt des Identifizierens von pathogenenen Muationen aus dem fötalen Genotyp in dem gemischten Genotyp umfasst:

Herausfiltern der polymorphen Stellen mit einer hohen Inzidenz in der humanen Population in einem fötalen Genotyp in dem Ziel-gemischten Genotyp an jeder SNP Stelle unter Erhalt von vorläufigen Kandidaten-Mutationsstellen;

Filtern von SNP Stellen von synonymen Mutationen und Nonsense-Mutationen und in nicht-konservierten Regionen auftretenden Mutationen aus den vorläufigen Kandidaten-Mutationsstellen unter Erhalt von Kandidaten-Mutationsstellen; und

Durchführen einer Literatur- und Datenbankrecherche nach Information in Zusammenhang mit einer monogenetischen Erkrankung an den Kandidaten-Mutationsstellen unter Erhalt der pathogenen Mutationen.

4. Vorrichtung zum Nachweis von Genmutationen, wobei die Vorrichtung umfasst:

ein Nachweismodul zum Durchführen von Hochdurchsatz-Sequenzierung von zellfreier DNA in mütterlichem peripheren Blut unter Erhalt von Sequenzierungsdaten;

ein Vergleichsmodul zum Vergleichen der Sequenzierungsdaten mit einem Referenzgenom unter Erhalt von SNP Stellen;

ein Bestimmungsmodul für einen Ziel-gemischten Genotyp zum Durchführen der gemischten Genotypisierung an jeder SNP Stelle mit einem Bayes Modell und einer anfänglichen fötalen Konzentration $f$ unter Erhalt eines gemischten Genotyps mit der maximalen Wahrscheinlichkeit unter sieben gemischten Genotypen an jeder SNP Stelle gemäß Anspruch 1, und das Festlegen des gemischten Genotyps mit der maximalen Wahrscheinlichkeit als der Ziel-gemischte Genotyp an jeder der SNP Stellen; und

ein Modul für das Absuchen von Mutationsstellen zum Identifizieren aus dem fötalen Genotyp in dem gemischten Genotyp Mutationen in dem Ziel-gemischten Genotyp an jeder der SNP-Stellen;

wobei der gemischte Genotyp Pseudo-Tetraploid Genotypen bedeutet, die zusammengesetzt sind aus den Genotypen der schwangeren Frau und des Föten, wobei der gemischte Genotyp ein Typ ausgewählt aus den Typen AAAA, AAAB, ABAA, ABAB, ABBB, BBAB und BBBB, wobei A ein Referenzallel an jeder der SNP Stellen bedeutet, und B ein mutiertes Allel an jeder der SNP Stellen bedeutet,

wobei die sieben Typen aufeinanderfolgend als Typ 1, Typ 2, Typ 3, Typ 4, Typ 5, Typ 6 und Typ 7 nummeriert werden.

5. Vorrichtung gemäß Anspruch 4, wobei das Modul zum Absuchen von Mutationsstellen umfasst:

ein Submodul zum Herausfiltern von Hochinzidenz polymorphen Stellen für das Herausfiltern von polymorphen Stellen mit hoher Inzidenz in der humanen Population in einem fötalen Genotyp in dem Ziel-gemischten Genotyp von jeder der SNP Stellen unter Erhalt der vorläufigen Kandidaten-Mutationsstellen;

ein Submodul zum Absuchen von Genmutationsstellen für das Filtern von SNP Stellen von synonymen Mutationen, nonsense Mutatione und in nicht-konservierten Bereichen auftretenden Mutationen aus den vorläufigen Kandidaten-Mutationsstellen unter Erhalt von Kandidaten-Mutationsstellen; und

ein Submodul zur Sichtung von Literatur- und klinischen Daten für das Durchführen einer Literatur- und Datenbankrecherche nach Information in Zusammenhang mit einer monogenetischen Erkrankung an den Kandidaten-Mutationsstellen unter Erhalt der pathogenen Mutationen.

**Revendications**

1. Procédé implémenté par ordinateur destiné à détecter des mutations dans un génome fœtal, sachant que le procédé comprend les étapes de :

   étape A, obtention de données de séquençage à partir d'un séquençage à haut débit d'ADN acellulaire dans du sang périphérique maternel ;
   étape B, alignement des données de séquençage avec celles d'un génome de référence pour obtenir des sites SNP ;
   étape C, mise en œuvre d'un génotypage mixte pour chacun des sites SNP moyennant un modèle bayésien et une concentration fœtale $f$ pour obtenir un génotype mixte à la probabilité maximale parmi sept génotypes mixtes pour chacun des sites SNP, et l'adoption du génotype mixte à la probabilité maximale en tant que génotype mixte cible de chacun des sites SNP, sachant que la mise en œuvre d'un génotypage mixte comprend les étapes de :
   calcul de

$$\varphi_j = \frac{P(G_{ij} \mid S_i)}{P(G_{ij*} \mid S_i)} = \frac{P(S_i \mid G_{ij})P(G_{ij})}{P(S_i \mid G_{ij*})P(G_{ij*})} \qquad (4)$$

   sachant que $\varphi_j$ représente le rapport de la probabilité du génotype mixte $G_j$ au i-ème site SNP à une probabilité du génotype mixte $G_j^*$ au i-ème site SNP à la condition $S_i$,
   sachant que P(S$_i$ | Gij) est calculé comme suit :

$$P(S_i \mid G_{ij}) = \binom{k+r-1}{r-1} \times f(b)^r \times (1-f(b))^k \qquad (7),$$

   sachant que r représente la survenance en nombre d'un allèle mutant au i-ème site SNP, k représente la survenance en nombre d'un allèle de référence au i-ème site SNP,
   sachant que f(b) se rapporte à la probabilité de l'allèle mutant dans le génotype mixte, lorsque le génotype mixte $G_{ij}$ est $G_{i1}$, la valeur de la $f(b)$ est 0 ; lorsque le génotype mixte $G_{ij}$ est $G_{i2}$, la valeur de la $f(b)$ est $f$/2 ;lorsque le génotype mixte $G_{ij}$ est $G_{i3}$, la valeur de la $f(b)$ est 0,5-$f$/2 ; lorsque le génotype mixte $G_{ij}$ est $G_{i4}$, la valeur de la $f(b)$ est 0,5 ; lorsque le génotype mixte $G_{ij}$ est $G_{i5}$, la valeur de la $f(b)$ est 0,5+$f$/2 ; lorsque le génotype mixte $G_{ij}$ est $G_{i6}$, la valeur de la $f(b)$ est 1-$f$/2 ; et lorsque le génotype mixte $G_{ij}$ est $G_{i7}$, la valeur de la $f(b)$ est 1 ;
   sachant que la $f$ représente la concentration fœtale,
   sachant que P(G$_{ij}$) est calculé comme suit :

$$\begin{cases} P(G' = AA) = (1-\theta)^2 \\ P(G' = AB) = 2\theta(1-\theta) \\ P(G' = BB) = \theta^2 \end{cases} \qquad (6)$$

   sachant que $\theta$ est la fréquence de mutation de population du i-ème site SNP, et identification du génotype mixte à probabilité de survenance maximale parmi les sept génotypes mixtes moyennant la formule

$$G = \arg\max(\varphi_j) \qquad (5),$$

   et
   enregistrement du génotype mixte à probabilité de survenance maximale en tant que le génotype mixte à probabilité maximale au i-ème site SNP ; et
étape D, identification, à partir du génotype fœtal dans le génotype mixte, de mutations pathogènes ;
sachant que le génotype mixte se rapporte au génotype pseudo-tétraploïde, qui est composé de génotypes de la femme enceinte et du fœtus, le génotype mixte est l'un quelconque de sept types, AAAA, AAAB, ABAA, ABAB, ABBB, BBAB, et BBBB, où A représente l'allèle de référence de chaque site SNP, et B représente l'allèle mutant de chaque site SNP, sachant que les sept types sont numérotés séquentiellement comme type 1, type 2, type 3, type 4, type 5, type 6 et type 7, et
sachant que le procédé n'inclut pas de séquençage séparé d'échantillons paternels et/ou maternels.

2. Le procédé selon la revendication 1, sachant que la concentration fœtale est calculée par itération comme suit :

étape 0 : pré-estimation que la concentration fœtale est une valeur définie qui est choisie de 5 à 20 % ;
itération :

étape 1 : inférence du génotype fœtal selon le génotypage mixte tel que défini dans la revendication 1, et calcul de la concentration fœtale $f'$ selon la $f(b)$ dans le génotype ;
étape 2 : calcul de $\Delta f$, sachant que $\Delta f$=D($f$-$f'$) ;
étape 3 : si $\Delta f$<$\varepsilon$, l'itération se termine, sachant que $\varepsilon$ est inférieur ou égal à 0,001 ; et
étape 4 : obtention de la concentration fœtale $f$=$f$(b) ;
sachant que la fonction D( ) représente une fonction de distance, qui mesure une différence entre deux variables ; et sachant que f(b) se rapporte à la probabilité de l'allèle mutant dans le génotype mixte, lorsque le génotype mixte $G_{ij}$ est $G_{i1}$, la valeur de la $f(b)$ est 0 ; lorsque le génotype mixte $G_{ij}$ est $G_{i2}$, la valeur de la $f(b)$ est $f$/2 ;lorsque le génotype mixte $G_{ij}$ est $G_{i3}$, la valeur de la $f(b)$ est 0,5-$f$/2 ; lorsque le génotype mixte $G_{ij}$ est $G_{i4}$, la valeur de la $f(b)$ est 0,5 ; lorsque le génotype mixte $G_{ij}$ est $G_{i5}$, la valeur de la $f(b)$ est 0,5+$f$/2 ; lorsque le génotype mixte $G_{ij}$ est $G_{i6}$, la valeur de la $f(b)$ est 1-$f$/2 ; et lorsque le génotype mixte $G_{ij}$ est $G_{i7}$, la valeur de la $f(b)$ est 1.

3. Le procédé selon la revendication 1, sachant que les étapes d'identification, à partir du génotype fœtal dans le génotype mixte, de mutations pathogènes comprend :

le filtrage des sites polymorphes à incidence élevée dans la population humaine dans un génotype fœtal dans le génotype mixte cible de chacun des sites SNP pour obtenir des sites de mutations candidats préliminaires ;
le filtrage de sites SNP de mutations synonymes et de mutations non-sens et de mutations survenant dans des régions non conservées, à partir de sites de mutations candidats préliminaires pour obtenir des sites de mutations candidats ; et
la mise en œuvre d'une recherche, dans la littérature et des bases de données, d'informations associées à une maladie monogénique sur les sites de mutations candidats pour obtenir les mutations pathogènes.

4. Dispositif destiné à détecter des mutations de gènes, sachant que le dispositif comprend :

un module de détection destiné à mettre en œuvre un séquençage à haut débit d'ADN acellulaire dans du sang périphérique maternel pour obtenir des données de séquençage ;
un module d'alignement destiné à aligner les données de séquençage avec une séquence génomique de référence pour obtenir des sites SNP ;
un module de détermination de génotype mixte cible destiné à mettre en œuvre un génotypage mixte sur chaque site SNP moyennant un modèle bayésien et une concentration fœtale $f$ initiale pour obtenir un génotype mixte à la probabilité maximale parmi sept génotypes mixtes de chaque site SNP selon la revendication 1, et l'adoption du génotype mixte à la probabilité maximale en tant que le génotype mixte cible de chacun des sites SNP ; et
un module de dépistage de site de mutations destiné à identifier, à partir du génotype fœtal dans le génotype mixte, des mutations pathogènes dans le génotype mixte cible de chacun des sites SNP ;
sachant que le génotype mixte se rapporte au génotype pseudo-tétraploïde, qui est composé de génotypes de la femme enceinte et du fœtus, le génotype mixte est l'un quelconque de sept types, AAAA, AAAB, ABAA, ABAB, ABBB, BBAB, et BBBB, où A représente un allèle de référence de chacun des sites SNP, et B représente un allèle mutant de chacun des sites SNP, sachant que les sept types sont numérotés séquentiellement comme type 1, type 2, type 3, type 4, type 5, type 6 et type 7.

5. Le dispositif selon la revendication 4, sachant que le module de dépistage de site de mutations comprend :

un sous-module de filtrage de sites polymorphes à incidence élevée destiné à filtrer des sites polymorphes à incidence élevée dans la population humaine dans un génotype fœtal dans le génotype mixte cible de chacun des sites SNP pour obtenir des sites de mutations candidats préliminaires ;

un sous-module de dépistage de sites de mutations de gènes destiné à filtrer des sites SNP de mutations synonymes, de mutations non-sens et de mutations survenant dans des régions non conservées, à partir des sites de mutations candidats préliminaires pour obtenir des sites de mutations candidats ; et

un sous-module d'examen de données de littérature et cliniques destiné à mettre en œuvre une recherche, dans la littérature et des bases de données, d'informations associées à une maladie monogénique sur les sites de mutations candidats pour obtenir les mutations pathogènes.

Step A: perform high-throughput sequencing on cell-free DNA extracted from pregnant women's peripheral blood to obtain sequencing data

Step B: align the sequencing data with reference genome to obtain SNP sites

Step C: perform mixed genotyping for each SNP site using Bayesian model and an initial fetal concentration $f$ to obtain a mixed genotype with the maximum probability among seven mixed genoTypes for each SNP site, and take the mixed genotype with the maximum probability as the target mixed genotype of each SNP site

Step D: identify the mutant alleles that cause the gene mutation of the fetuses according to the genotype of the fetus in the target mixed genotypes

Figure 1

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2015249846 A2
- US 20130123120 A1
- EP 17888263 A
- CN 201611270836
- CN 2017118213 W

**Non-patent literature cited in the description**

- **LENCH N ; BARRETT A ; FIELDING S et al.** The clinical implementation of non-invasive prenatal diagnosis for single-gene disorders: challenges and progress made [J. *Prenatal diagnosis,* 2013, vol. 33 (6), 555-562